# EUROPÄISCHE PATENTSCHRIFT

(11) **EP 2 869 749 B1**
(45) Veröffentlichungstag und Bekanntmachung des Hinweises auf die Patenterteilung: **18.09.2019**
(21) Anmeldenummer: 13745597.8
(22) Anmeldetag: 05.07.2013
(51) Int. Cl.: A61B 1/00, A61B 1/008, A61B 17/00, A61B 1/01, A61B 17/34, A61B 1/313

(54) **MEDIZINISCHES INSTRUMENT ZUM VERSCHWENKEN EINES SOLCHEN MEDIZINISCHEN INSTRUMENTS**
MEDICAL INSTRUMENT FOR PIVOTING SUCH A MEDICAL INSTRUMENT
INSTRUMENT MÉDICAL DE PIVOTEMENT D'UN TEL INSTRUMENT MÉDICAL

(30) Priorität: 06.07.2012 DE 102012211886
(43) Veröffentlichungstag der Anmeldung: 13.05.2015
(73) Patentinhaber: Karl Storz SE & Co. KG, 78532 Tuttlingen (DE)
(72) Erfinder: BLASE, Bastian, 10439 Berlin (DE); ERBER, Felix, 10559 Berlin (DE)
(74) Vertreter: Maikowski & Ninnemann Patentanwälte Partnerschaft mbB
(86) Internationale Anmeldenummer: PCT/EP2013/064267
(87) Internationale Veröffentlichungsnummer: WO 2014/006186

(56) Entgegenhaltungen:
- WO-A1-2007/089676
- US-A- 2 079 233
- US-A- 5 467 763
- US-A1- 2004 225 305
- US-A1- 2007 260 114
- US-A1- 2008 046 122
- US-A1- 2008 194 911
- US-A1- 2010 262 180

## Beschreibung

Die Erfindung betrifft ein medizinisches Instrument zum Führen eines Werkzeugs in einen Operationsraum nach dem Oberbegriff des Anspruchs 1 und ein Verfahren zum Verschwenken eines solchen medizinischen Instruments.

Ein solches medizinisches Instrument umfasst einen Schaft, der ein erstes Schaftsegment und ein zweites Schaftsegment, die gelenkig miteinander verbunden sind, aufweist, und ein Verstellelement, das entlang einer Verstellrichtung längsverschieblich in einer inneren Bohrung des ersten oder zweiten Schaftsegments angeordnet und verstellbar ist, um das erste Schaftsegment und das zweite Schaftsegment relativ zueinander zu verschwenken. Des Weiteren umfasst ein solches medizinisches Instrument ein Verbindungselement, das über eine erste Gelenkverbindung gelenkig mit dem Verstellelement und über eine zweite Gelenkverbindung gelenkig mit dem anderen von erstem und zweitem Schaftsegment verbunden ist. Dabei ist vorgesehen, dass durch Verstellen des Verstellelements entlang der Verstellrichtung das erste Schaftsegment und das zweite Schaftsegment zueinander verschwenkbar sind.

Ein solches medizinisches Instrument kann beispielsweise als Endoskop oder chirurgisches bzw. minimal-invasives Instrument eingesetzt werden, dass durch eine Körperöffnung hindurch in einen Körper eingeführt wird, um im Rahmen eines minimal-invasiven Eingriffs eine Operation im Körperinneren vorzunehmen. Durch den Schaft werden hierbei medizinische Werkzeuge, beispielsweise eine Zange, ein Greifarm, ein Schneidwerkzeug oder dergleichen in das Körperinnere eingeführt, um mittels des Werkszeugs die erforderliche Handlung vorzunehmen. Alternativ kann sich ein Werkzeug (auch als Manipulator bezeichnet) fest verbaut an der Spitze des Schafts befinden.

Der Schaft weist mindestens zwei Schaftsegmente auf, die gelenkig miteinander verbunden sind. Auf diese Weise wird möglich, durch Verschwenken der Schaftsegmente zueinander die Führungsbahn für ein Werkzeug zu ändern und das Werkzeug mittels des Schafts auch hin zu schwer zugänglichen Orten zu führen.

In der US 2004/0225305 wird ein medizinisches Instrument beschrieben mit zwei Schaftsegmenten, die über ein Verbindungselement gelenkig miteinander verbunden sind. Durch verschieben des ersten Schaftsegments, wird das zweite Schaftsegment verschwenkt.

In der US 5,467,763 ist ein Operationsbesteck beschrieben, welches einen Retraktor mit wenigstens zwei Schaftsegmenten umfassen kann. In einem längserstrecktem Zustand liegen ein zweites Schaftsegment und ein Verbindungselement sowie ein Verstellelement innerhalb eines ersten Schaftsegments. Durch Verschieben des Verstellelements, verschwenken das zweite Schaftsegment und das Verbindungselement bezüglich des ersten Schaftsegments und liegen zumindest teilweise außerhalb des zweiten Schaftsegments.

Die US 2008/0046122 A1 zeigt ein Kontrollsystem für ein Roboteroperationsbesteck. Das Kontrollsystem weist eine Eingabevorrichtung auf, welche rotatorische Bewegungen und auch Zusammendrückbewegungen eines Griffs erfassen und weiterleiten kann. Bei der Zusammendrückbewegung, bzw. Öffnen des Griffs werden zwei Schaftsegmente zueinander verschwenkt, wobei ein Teil des Griffs ein zweites Schaftsegment ausbildet. Das Verschwenken erfolgt durch Verschieben eines Verstellelements, welches über ein Verbindungselement gelenkig mit dem zweiten Schaftsegment verbunden ist.

Die US 2007/0260114 A1 offenbart ein endoskopisches Instrument mit einer X-förmigen Verbindungsstruktur, die einen Einführungsabschnitt und ein anpassbares Glied zueinander verschwenkt. Dabei ist ein Stabelement zum Bewirken der Verschwenkung verschiebbar innerhalb des Einführungsabschnitts geführt.

Bei einem aus der US 6,099,464 bekannten medizinischen Instrument ist ein flexibler Schaft vorgesehen, der durch Aufbringen einer Zugkraft im Bereich seiner Spitze gekrümmt werden kann.

Aus der US 6,036,636 ist ein medizinisches Instrument mit einem Schaft bekannt, der zwei gelenkig miteinander verbundene Schaftsegmente aufweist. An dem einen Schaftsegment greift ein Draht an, der in dem anderen Schaftsegment geführt ist derart, dass durch Zug an dem Draht das eine Schaftsegment relativ zu dem anderen Schaftsegment verschwenkt werden kann.

Bei einem aus der EP 1 972 259 A2 bekannten medizinischen Instrument weist ein Schaft zwei Schaftsegmente auf, die gelenkig miteinander verbunden sind. An dem einen Schaftsegment greift ein Betätigungselement in Form eines Drahtes an, der mit einem Hebel an dem anderen Schaftsegment gekoppelt ist derart, dass durch Verschwenken des Hebels das eine Schaftsegment relativ zu dem anderen Schaftsegment verstellt und auf diese Weise der Schaft im Bereich seiner Spitze abgeknickt werden kann.

Sollen mittels eines solchen medizinischen Instruments auch schwer zugängliche Orte im Körperinneren erreichbar sein oder soll eine komplexe Operationstechnologie wie ein so genannter "Single Port"-Eingriff zum Einsatz kommen, sind mehrere gelenkig miteinander verbundene Schaftsegmente erforderlich, die zur Führung des medizinischen Instruments hin zum Operationsort zueinander verschwenkt werden können. Gelenke bilden bei solchen Schaftanordnungen jedoch regelmäßig Schwachstellen aus, weil die Gelenke auf kleinem Raum verwirklicht werden müssen und somit filigran ausgebildet sind und zudem Durchführungen für Werkzeuge oder Daten- und Versorgungsleitungen oder auch Lichtleiter aufweisen müssen. Weil bei verschwenktem Schaftsegment Kräfte über einen Hebelarm auf ein zugeordnetes Gelenk wirken, können die Lastkräfte und -momente am Gelenk groß sein.

Es besteht somit ein Bedürfnis für ein medizinisches Instrument mit einem Schaft, der mehrere gelenkig miteinander verbundene Schaftsegmente aufweist und bei dem wirkende Kräfte in vorteilhafter Weise abgestützt sind.

Aufgabe der vorliegenden Erfindung ist es, ein medizinisches Instrument sowie ein Verfahren zum Verschwenken eines medizinischen Instruments zur Verfügung zu stellen, die eine einfache Handhabung bei gleichzeitig vorteilhafter Kraftabstützung ermöglichen.

Diese Aufgabe wird durch einen Gegenstand mit den Merkmalen des Anspruchs 1 gelöst.

Die vorliegende Erfindung geht von dem Gedanken aus, durch Verwendung eines Verbindungselements, das gelenkig mit beiden Schaftsegmenten gekoppelt ist, ein Dreigelenk zu schaffen, bei dem eine erste Seite durch das erste Schaftsegment, eine zweite Seite durch das zweite Schaftsegment und eine dritte Seite durch das Verbindungselement gebildet ist. Das erste Schaftsegment, das zweite Schaftsegment und das Verbindungselement spannen in einem Zustand, in dem die Schaftsegmente zueinander verschwenkt sind, ein Dreieck auf, bei dem das Verbindungselement das erste Schaftsegment und das zweite Schaftsegment gegeneinander abstützt.

Das Verschwenken der Schaftsegmente zueinander wird über das Verbindungselement gesteuert. Dazu ist das Verbindungselement einerseits mit einem Schaftsegment gelenkig gekoppelt und anderseits gelenkig an einem an dem anderen Schaftsegment verschiebbar angeordneten Verstellelement angeordnet derart, dass durch Verschieben des Verstellelements an dem zugeordneten Schaftsegment die Schaftsegmente zueinander verschwenkt werden können. Wird das Verstellelement von einem Gelenk, das das erste Schaftsegment und das zweite Schaftsegment gelenkig miteinander verbindet, in axialer Richtung entfernt, so werden die beiden Schaftsegmente beispielsweise aus einem Längserstreckungszustand, in dem sie im Wesentlichen miteinander fluchten, zueinander verschwenkt, so dass sich ein zwischen den Schaftsegmenten aufgespannter Winkel verkleinert.

Das Verbindungselement ist gelenkig mit dem einen Schaftsegment und gelenkig mit dem an dem anderen Schaftsegment geführten Verstellelement gekoppelt. Denkbar ist in diesem Zusammenhang auch, an beiden Schaftsegmenten jeweils ein Verstellelement zu führen, mit dem das Verbindungselement jeweils gelenkig gekoppelt ist, so dass das Verbindungselement an keinem Schaftsegment ortsfest angelenkt ist. Durch Vorsehen zweier solcher Verstellelement, die gegensätzlich zueinander verstellbar sind, kann für ein schnelles Verschwenken der Schaftsegmente zueinander gesorgt werden.

Dadurch, dass das Verschwenken der Schaftsegmente zueinander über das Verbindungselement bewirkt wird, wird ein Hebelgetriebe geschaffen, dass sich mit zunehmender Abwinkelung der Schaftsegmente zueinander verstärkend abstützt, indem das Verbindungselement als Strebe zwischen den Schaftsegmenten wirkt und eine vorteilhafte Abstützung bereitstellt.

Das Verstellelement ist vorteilhafterweise zum Verstellen entlang der Verstellrichtung motorisch angetrieben und kann beispielsweise mit einer Spindel eines Spindelgetriebes in Eingriff stehen, mittels dessen eine Längsverstellung des Verstellelements entlang seiner Verstellrichtung bewirkt werden kann. Der motorische Antrieb kann zum motorischen Verstellen des Verstellelements ausgelegt sein. Denkbar ist aber auch, dass ein motorischer Antrieb kraftunterstützend wirkt derart, dass ein manuelles Verstellen des Verstellelements nach Art eines Servoantriebs motorisch unterstützt wird.

Das Verbindungselement ist als halbzylindrisch geformtes Halbrohr ausgebildet. Bei einem Halbrohr handelt es sich um ein entlang seiner Längsachse geteiltes halbes Rohr, dass zu einer Seite hin gewölbt und zur anderen Seite hin entsprechend geöffnet ist. In einem Längserstreckungszustand, in dem die Schaftsegmente im Wesentlichen kollinear miteinander fluchten, liegt das als Halbrohr geformte Verbindungselement entsprechend an den Schaftsegmenten an und umgreift diese zumindest abschnittsweise, so dass das Verbindungselement nach außen hin nicht wesentlich von den Schaftsegmenten vorsteht und ein im Wesentlichen zylindrischer Schaft geschaffen wird, der in einfacher Weise durch eine Körperöffnung, in das Körperinnere eines Patienten eingeführt werden kann.

Das als Halbrohr ausgebildete Verbindungselement weist eine innere Anlagefläche auf, entsprechend der inneren, zylindrischen Mantelfläche des Halbrohrs, die gleichzeitig einen Anschlag für die Schaftsegmente in Längserstreckungszustand bereitstellt derart, dass die Schaftsegmente nicht über den Längserstreckungszustand hinaus zueinander verschwenkt werden können.

Durch Verstellen des Verstellelements an dem einen der Schaftsegmente können die Schaftsegmente aus dem Längserstreckungszustand heraus zueinander verschwenkt und in einen verschwenkten Zustand überführt werden. Beim Überführen vom Längserstreckungszustand in einen verschwenkten Zustand gelangen die Schaftsegmente außer Anlage mit der inneren Anlagenfläche des Verbindungselements, so dass die Schaftsegmente und das Verbindungselement ein Dreieck aufspannen, dessen Dreiecksfläche sich in Abhängigkeit der Schwenkstellung der Schaftsegmente zueinander verändert.

Die erste Gelenkverbindung und die zweite Gelenkverbindung liegen in dem Längserstreckungszustand auf einer parallel zur Verstellrichtung gerichteten Linie, die zum Beispiel der Längsachse der im Längserstreckungszustand miteinander fluchtenden Schaftsegmente entsprechen kann. Vorteilhafterweise liegt dann ein Gelenk, das das erste Schaftsegment und das zweite Schaftsegment gelenkig miteinander verbindet, quer zur Verstellrichtung um einen Abstand von der Linie beabstandet, so dass die Gelenkverbindungen, über die das Verbindungselement gelenkig mit den Schaftsegmenten gekoppelt ist, und das Gelenk, mit dem die Schaftsegmente gelenkig miteinander verbunden sind, nicht auf einer gemeinsamen Linie liegen. Das Gelenk, mit dem die Schaftsegmente gelenkig miteinander verbunden sind, ist hierbei in eine von dem Verbindungselement weg weisende Richtung von der die Gelenkverbindungen miteinander verbindenden Linie beabstandet, was dazu führt, dass bei einem Verschieben des Verstellelements die Schaftsegmente ohne weiteres aus ihrem Längserstreckungszustand heraus zueinander verschwenkt werden können und das Verschieben des Verstellelements immer ein Moment um das die Schaftsegmente miteinander verbindende Gelenk bewirkt.

Das Verstellelement kann beispielsweise längsverschieblich in einer inneren Bohrung des zugeordneten Schaftsegments geführt sein. In der inneren Bohrung kann es dann beispielsweise mit einer Spindel eines Spindelantriebs in Eingriff stehen derart, dass angetrieben durch die Spindel das Verstellelement längs in der Bohrung des zugeordneten Schaftsegments verstellt werden kann. In diesem Fall greift die beispielsweise durch Zapfen verwirklichte erste Gelenkverbindung durch ein entlang der Längsrichtung erstrecktes Langloch an dem Schaftsegment, um außerhalb des Schaftsegments eine Kopplung des Verstellelements mit dem Verbindungselement herzustellen.

Alternativ kann das Verstellelement auch als Hülse ausgebildet sein, die längsverschieblich außen an einem Schaftabschnitt des zugeordneten Schaftsegments angeordnet ist. In diesem Fall ist das Verstellelement außerhalb des Schaftsegments geführt und kann längs entlang des Schaftabschnitts des Schaftsegments bewegt werden.

Soll das medizinische Instrument bei einem Eingriff verwendet werden, bei dem ein schwer zugänglicher Ort im Körperinneren erreicht werden soll oder bei dem mehrere Instrumente über eine einzige Öffnung beispielsweise in der Bauchdecke eines Patienten hin zu einem Operationsort geführt werden sollen (so genannter "Single Port"-Eingriff), ist es erforderlich, den Schaft in mehrere Schaftsegmente zu teilen, die in unterschiedliche Richtungen zueinander verschwenkt werden können. In einem solchen Fall kann der Schaft beispielsweise ein weiteres, drittes Schaftsegment aufweisen, das gelenkig mit dem zweiten Schaftsegment verbunden ist und somit an das zweite Schaftsegment anschließt. Das erste Schaftsegment, das zweite Schaftsegment und das dritte Schaftsegment fluchten in einem Längserstreckungszustand des Schafts im Wesentlichen kollinear miteinander und sind in einem verschwenkten Zustand in einer gemeinsamen Schwenkebene zueinander verschwenkt. Hierunter ist zu verstehen, dass die Schwenkachsen, über die die Schaftsegmente zueinander verschwenkbar sind, parallel zueinander gerichtet sind, so dass ein Verschwenken der Schaftsegmente immer in einer gemeinsamen Ebene erfolgt und die Schaftsegmente diese Ebene nicht verlassen.

Vorteilhafterweise sind ein das erste Schaftsegment und das zweite Schaftsegment verbindendes, erstes Gelenk und ein das zweite Schaftsegment und das dritte Schaftsegment verbindendes, zweites Gelenk in dem Längserstreckungszustand in unterschiedliche Richtungen quer zu einer längs entlang der Schaftsegmente erstreckten Mittenlinie versetzt. Auf diese Weise kann erreicht werden, dass über geeignete Verbindungselemente die Schaftsegmente in unterschiedliche Richtungen zueinander verschwenkt werden können, so dass das zweite Schaftsegment beispielsweise nach außen relativ zum ersten Schaftsegment verschwenkt werden kann, um dann das dritte Schaftsegment vom zweiten Schaftsegment zurück nach innen zu schwenken. Auf diese Weise kann beispielsweise bei einem "Single Port"-Eingriff, bei dem unterschiedliche Instrumente über eine einzige Öffnung in das Körperinnere eines Patienten geführt werden, das Instrument dem Operationsort von außen angenähert werden, ohne dass sich die unterschiedlichen Instrumente in die Quere kommen.

Zum Steuern der Bewegung der drei Schaftsegmente zueinander kann ein weiteres, zweites Verbindungselement vorgesehen sein, dass zwischen dem zweiten Schaftsegment und dem dritten Schaftsegment wirkt und das Verschwenken des zweiten Schaftsegments relativ zum dritten Schaftsegment steuert.

Für ein solches zweites Verbindungselement können, im Zusammenspiel mit dem ersten Verbindungselement, unterschiedliche Konfigurationen vorgesehen sein.

So kann in einer ersten Variante das zweite Verbindungselement getrennt von dem zwischen dem ersten Schaftsegment und dem zweiten Schaftsegment wirkenden, ersten Verbindungselement ausgebildet sein. Das zweite Verbindungselement ist in diesem Fall über eine dritte Gelenkverbindung mit einem von zweitem und drittem Schaftsegment und über eine vierte Gelenkverbindung mit einem an dem anderen von zweitem und drittem Schaftsegment verstellbar angeordneten, zweiten Verstellelement verbunden. Auf diese Weise kann das Verschwenken des zweiten Schaftsegments und des dritten Schaftsegments zueinander über das zweite Verbindungselement völlig unabhängig von dem Verschwenken des ersten Schaftsegments und des zweiten Schaftsegments zueinander gesteuert werden, indem das zweite Verstellelement beispielsweise in motorisch angetriebener Weise verstellt wird, um das zweite Schaftsegment und das dritte Schaftsegment relativ zueinander zu verschwenken. Es ergibt sich eine große Flexibilität zum Verschwenken der Schaftsegmente zueinander, bei gegebenenfalls aufwändigerer elektronischer Steuerung sowie bedientechnischer Handhabung für einen Nutzer.

Alternativ kann in einer zweiten Variante das Verschwenken des zweiten und dritten Schaftsegments zueinander auch gekoppelt sein an das Verschwenken des ersten und zweiten Schaftsegments zueinander, so dass ein unabhängiges Verschwenken des dritten Schaftsegments nicht möglich ist. Eine Möglichkeit dies zu erreichen ist, bei Verwendung von getrennten Verbindungselementen die Steuerung des zweiten Verstellelements elektronisch zu koppeln mit der Steuerung des ersten Verstellelements. Die Kopplung der Verschwenkbewegungen erfolgt somit auf elektronische Weise.

In einer dritten Variante ist auch denkbar, das erste Verbindungselement und das zweite Verbindungselement starr miteinander zu verbinden, indem das erste Verbindungselement und das zweite Verbindungselement beispielsweise einstückig ausgebildet sind. Das erste Verbindungselement koppelt das erste Schaftsegment und das zweite Schaftsegment miteinander, während das zweite Verbindungselement das zweite Schaftsegment und das dritte Schaftsegment miteinander verbindet. In diesem Fall kann ein erstes Verstellelement beispielsweise verstellbar an dem erstem Schaftsegment und ein zweites Verstellelement verstellbar an dem zweiten oder dem dritten Schaftsegment angeordnet sein. Das erste Verbindungselement steht über eine erste Gelenkverbindung gelenkig mit dem ersten Verstellelement und über eine zweite Gelenkverbindung gelenkig mit dem zweiten Schaftsegment oder dem am zweiten Schaftsegment angeordneten zweiten Verstellelement in Verbindung, während das zweite Verbindungselement über die zweite Gelenkverbindung gelenkig mit dem zweiten Schaftsegment bzw. dem am zweiten Schaftsegment geführten zweiten Verstellelement und über eine dritte Gelenkverbindung gelenkig mit dem am dritten Schaftsegment geführten zweiten Verstellelement oder dem dritten Schaftsegment gekoppelt ist. Dadurch, dass das erste Verbindungselement und das zweite Verbindungselement starr miteinander verbunden sind und somit ein einheitliches, gemeinsames Verbindungselement zur Kopplung der drei Schaftsegmente miteinander ausbilden, kann durch Antreiben eines einzigen Verstellelements ein Verschwenken der drei Schaftsegmente zueinander in vordefinierter Weise vorgenommen werden. Das zweite Verstellelement kann in diesem Fall lediglich passiv mitlaufen, ohne selbst motorisch angetrieben zu sein, wobei auch denkbar ist, einen zusätzlichen Antrieb auch für das zweite Verstellelement vorzusehen, der elektronisch in Abhängigkeit von der Bewegung des ersten Verstellelements gesteuert wird.

Sind das erste Verbindungselement und das zweite Verbindungselement in starrer Weise miteinander verbunden, so ist die Kinematik der Schaftsegmente vorteilhafterweise so ausgestaltet, dass bei einem Verstellen des Verstellelements beispielsweise das zweite Schaftsegment relativ zum ersten Schaftsegment nach außen verschwenkt wird, während das dritte Schaftsegment relativ zum zweiten Schaftsegment nach innen gelenkt wird, so dass sich näherungsweise eine S-Form ergibt, mittels derer das Instrument einem Operationsort von außen angenähert werden kann.

Das erste Verbindungselement und das zweite Verbindungselement sind vorteilhafterweise jeweils als Halbrohr ausgebildet, wobei die Verbindungselemente vorteilhafterweise in zueinander entgegengesetzen Richtungen geöffnet sind. Im Längserstreckungszustand liegt das erste Verbindungselement somit von der einen Seite an den zugeordneten Schaftsegmenten an, während das andere, zweite Verbindungselement an einer diametral gegenüber liegenden Seite der zugeordneten Schaftsegmente anliegt. Bei einem Verschwenken der Schaftsegmente werden die die Schaftsegmente miteinander verbindenden Gelenke in unterschiedliche Richtungen von dem jeweils zugeordneten Verbindungselement entfernt, so dass sich eine unterschiedlich gerichtete Verschwenkbewegung der Schaftsegmente zueinander ergibt.

In einer vorteilhaften Ausgestaltung sind das zweite Schaftsegment und/oder die Verbindungselemente in dem Längserstreckungszustand zwischen dem ersten Schaftsegment und dem dritten Schaftsegment aufgenommen und zumindest abschnittsweise von dem ersten Schaftsegment und dem dritten Schaftsegment eingefasst. In dem Längserstreckungszustand sind die Schaftsegmente sowie auch die Verbindungselemente somit in kompakter Weise einander angenähert, wobei das erste Schaftsegment und das dritte Schaftsegment beispielsweise jeweils eine Aufnahmeöffnung in einem nach Art eines Halbrohrs geformten Abschnitt aufweisen, in der das zweite Schaftsegment und/oder die Verbindungselemente in dem Längserstreckungszustand einliegen.

Das erste Schaftsegment und das dritte Schaftsegment können hierbei beispielsweise spiegelsymmetrisch zueinander geformt sein und im Längserstreckungszustand derart aneinander angesetzt sein, dass sie eine im Wesentlichen geschlossene Hülle bilden, in der das zweite Schaftsegment und die Verbindungselemente eingefasst sind. Im Längserstreckungszustand kann der Schaft somit in einfacher Weise durch einen Port beispielsweise in einen Bauchraum eines Patienten eingeführt werden, um dann, nach Einführung in den Port, aufgeschwenkt und in einen betriebsgemäßen Zustand zum Durchführen eines medizinischen Eingriffs gebracht zu werden.

In dem Längserstreckungszustand können das erste Schaftsegment und das zweite Schaftsegment und gleichermaßen auch das zweite Schaftsegment und das dritte Schaftsegment paarweise jeweils einen Winkel von 0°C zueinander aufweisen (bezogen auf die Längsachsen der Schaftsegmente), so dass das zweite Schaftsegment hin zum ersten Schaftsegment und das dritte Schaftsegment hin zum zweiten Schaftsegment geklappt sind und dabei das zweite Schaftsegment sowie auch die Verbindungselemente zwischen dem ersten Schaftsegment und dem dritten Schaftsegment zu liegen kommen.

In dem Längserstreckungszustand ist der Schaft somit verkürzt. Durch Verstellen des Schafts aus seinem Längserstreckungszustand heraus wird dann das dritte Schaftsegment von dem ersten Schaftsegment durch Ausschwenken des zweiten Schaftsegments entfernt, wobei sich der Winkel zwischen dem ersten Schaftsegment und dem zweiten Schaftsegment sowie auch zwischen dem zweiten Schaftsegment und dem dritten Schaftsegment vergrößert.

Bei Verstellen des Schafts aus seinem Längserstreckungszustand heraus kann vorgesehen sein, dass das dritte Schaftsegment im Wesentlichen quer vom ersten Schaftsegment entfernt wird und dabei eine im Wesentlichen parallele Lagebeziehung zum ersten Schaftsegment beibehält.

Denkbar und möglich ist aber auch, dass durch geeignete Auswahl der Längen des ersten Verbindungselements zwischen dem ersten Schaftsegment und dem zweiten Schaftsegment sowie des zweiten Verbindungselements zwischen dem zweiten Schaftsegment und dem dritten Schaftsegment eine Trajektorie definiert wird, im Rahmen derer bei Verstellen des Schafts aus einem Längserstreckungszustand heraus zunächst das dritte Schaftsegment ausgeschwenkt wird, um dann bei weiterem Verstellen das dritte Schaftsegment wieder einzuschwenken, so dass ein vom zweiten Schaftsegment abliegender Kopf des dritten Schaftsegments wiederum hin zu einer Achse weist, entlang derer sich das erste Schaftsegment erstreckt. Auf diese Weise kann ein Werkzeug in vorteilhafter Weise hin zu einem Operationsort geführt werden, um am Operationsort einen gewünschten Eingriff durchzuführen.

In einer anderen Ausführungsform können das zweite Schaftsegment und das Verbindungselement auch jeweils als Halbrohr ausgebildet sein, wobei in einem Längserstreckungszustand das erste Schaftsegment und/oder das dritte Schaftsegment zumindest abschnittsweise in dem zweiten Schaftsegment oder in dem Verbindungselement einliegen. Das erste Schaftsegment, das zweite Schaftsegment, das dritte Schaftsegment und ein das erste Schaftsegment mit dem dritten Schaftsegment koppelndes Verbindungselement können in diesem Fall beispielsweise ein Viergelenk ausbilden. Eine Kopplung des Verbindungselements mit dem zweiten Schaftsegment entfällt hierbei.

Bei dieser Ausführungsform ist das Verbindungselement vorteilhafterweise gelenkig mit einem längsverschieblich am ersten Schaftsegment geführten Verstellelement verbunden und gelenkig, aber ortsfest an das dritte Schaftsegment angebunden. Das zweite Schaftsegment verbindet das erste Schaftsegment und das dritte Schaftsegment miteinander und ist hierbei jeweils gelenkig, aber ortsfest mit dem ersten Schaftsegment und dem dritten Schaftsegment gekoppelt. Um dabei eine Bewegungstrajektorie zum Verstellen des dritten Schaftsegments relativ zum ersten Schaftsegment festzulegen, ist zusätzlich ein Hebelelement vorgesehen, das sich zwischen dem Verbindungselement und dem ersten Schaftsegment erstreckt und dazu gelenkig und ortsfest mit dem Verbindungselement und dem ersten Schaftsegment verbunden ist. Bei einem Verstellen des Verstellelements wird somit das Verbindungselement verschwenkt, wobei die Bewegung des Verbindungselements festgelegt ist durch die Kopplung des Verbindungselements über das Hebelelement mit dem ersten Schaftsegment. Dem Verbindungselement folgend werden dann auch das zweite Schaftsegment und das dritte Schaftsegment verschwenkt.
Darunter, dass eine Verbindung gelenkig, aber ortsfest ist, ist vorliegend zu verstehen, dass ein Gelenk zur Verbindung zweier Elemente ortsfest an beiden Elementen, also nicht verschieblich zu einem der Elemente, angeordnet ist.

Die Aufgabe wird auch durch ein Verfahren zum Verschwenken eines medizinischen Instruments zum Führen eines Werkzeugs in einen Operationsraum gelöst. Das medizinische Instrument weist einen Schaft mit einem ersten Schaftsegment und einem zweiten Schaftsegment, die gelenkig miteinander verbunden sind, und ein Verstellelement, das entlang einer Verstellrichtung längsverschiebbar an einem von erstem und zweiten Schaftsegment angeordnet und verstellbar ist, um das erste Schaftsegment und das zweite Schaftsegment relativ zueinander zu verschwenken, auf. Dabei ist vorgesehen, dass ein Verbindungselement über eine erste Gelenkverbindung gelenkig mit dem Verstellelement und über eine zweite Gelenkverbindung gelenkig mit dem anderen von erstem und zweitem Schaftsegment verbunden ist und durch Verstellen des Verstellelements entlang der Verstellrichtung das erste Schaftsegment und das zweite Schaftsegment zueinander verschwenkt werden.

Die vorangehend für das medizinische Instrument beschriebenen Vorteile und vorteilhaften Ausgestaltungen finden analog auch auf das Verfahren Anwendung.

Der der Erfindung zugrunde liegende Gedanke soll nachfolgend anhand der in den Figuren dargestellten Ausführungsbeispiele näher erläutert werden. Es zeigen:
- Fig. 1A: eine Ansicht eines ersten Ausführungsbeispiels eines medizinischen Instruments mit einem zwei Schaftsegmente aufweisenden Schaft, in einem Längserstreckungszustand;
- Fig. 1B: eine Ansicht der Anordnung gemäß Fig. 1A, in einem verschwenkten Zustand;
- Fig. 2A: eine Ansicht eines medizinischen Instruments mit einem drei Schaftsegmente aufweisenden Schaft, in einem Längserstreckungszustand;
- Fig. 2B: eine Ansicht der Anordnung gemäß Fig. 2A, in einem verschwenkten Zustand;
- Fig. 3A: eine Ansicht eines weiteren Ausführungsbeispiels eines medizinischen Instruments mit einem drei Schaftsegmente aufweisenden Schaft, in einem Längserstreckungszustand;
- Fig. 3B: eine Ansicht der Anordnung gemäß Fig. 3A, in einem verschwenkten Zustand,
- Fig. 4A: eine Ansicht eines weiteren Ausführungsbeispiels eines medizinischen Instruments mit einem drei Schaftsegmente aufweisenden Schaft, in einem Längserstreckungszustand;
- Fig. 4B: eine Ansicht der Anordnung gemäß Fig. 4A, in einem verschwenkten Zustand;
- Fig. 5A: eine Ansicht eines weiteren Ausführungsbeispiels eines medizinischen Instruments mit einem drei Schaftsegmente aufweisenden Schaft, in einem Längserstreckungszustand;
- Fig. 5B: eine Ansicht der Anordnung gemäß Fig. 5A, in einem verschwenkten Zustand;
- Fig. 6A: eine Ansicht eines weiteren Ausführungsbeispiels eines medizinischen Instruments mit einem drei Schaftsegmente aufweisenden Schaft, in einem Längserstreckungszustand;
- Fig. 6B: eine Ansicht der Anordnung gemäß Fig. 6A, in einem verschwenkten Zustand;
- Fig. 7A: eine Ansicht eines weiteren Ausführungsbeispiels eines medizinischen Instruments mit einem drei Schaftsegmente aufweisenden Schaft, in einem Längserstreckungszustand;
- Fig. 7B: eine Ansicht der Anordnung gemäß Fig. 7A, in einem verschwenkten Zustand;
- Fig. 8: eine Ansicht eines als Halbrohr ausgebildeten Verbindungselements;
- Fig. 9A: eine perspektivische Ansicht eines anderen Ausführungsbeispiels eines Verbindungselements;
- Fig. 9B: eine Draufsicht auf das Verbindungselement gemäß Fig. 9A;
- Fig. 9C: eine Seitenansicht des Verbindungselements gemäß Fig. 9A;
- Fig. 10: eine Seitenansicht eines weiteren Ausführungsbeispiels eines medizinischen Instruments mit einem drei Schaftsegmente aufweisenden Schaft, in einem verschwenkten Zustand;
- Fig. 11A: eine Ansicht des Ausführungsbeispiels gemäß Fig. 10 in einem Längserstreckungszustand;
- Fig. 11B: eine Ansicht des medizinischen Instruments bei einem Verschwenken;
- Fig. 11C: eine Ansicht des medizinischen Instruments in einem verschwenkten Zustand;
- Fig. 12A: eine Seitenansicht der Anordnung gemäß Fig. 11A;
- Fig. 12B: eine Seitenansicht der Anordnung gemäß Fig. 11B;
- Fig. 12C: eine Seitenansicht der Anordnung gemäß Fig. 11C;
- Fig. 13: eine perspektivische Ansicht eines weiteren Ausführungsbeispiels eines medizinischen Instruments, in einem verschwenkten Zustand;
- Fig. 14A: eine Seitenansicht des medizinischen Instruments in einem Längserstreckungszustand;
- Fig. 14B: eine Seitenansicht des medizinischen Instruments beim Verschwenken;
- Fig. 14C: eine weitere Seitenansicht des medizinischen Instruments beim Verschwenken;
- Fig. 14D: eine Seitenansicht des medizinischen Instruments in einem verschwenkten Zustand;
- Fig. 15: eine perspektivische Ansicht des medizinischen Instruments bei einem Verschwenken;
- Fig. 16: eine Ansicht in den Schaft des medizinischen Instruments hinein;
- Fig. 17: eine perspektivische Ansicht des medizinischen Instruments in einem verschwenkten Zustand;
- Fig. 18A: eine perspektivische, teilweise transparente Ansicht eines weiteren Ausführungsbeispiels eines medizinischen Instruments mit einem drei Schaftsegmente aufweisenden Schaft, in einem verschwenkten Zustand;
- Fig. 18B: die Ansicht gemäß Fig. 18A, jedoch nicht transparent;
- Fig. 19A: eine Seitenansicht des medizinischen Instruments in einem Längserstreckungszustand;
- Fig. 19B: eine Seitenansicht des medizinischen Instruments bei einem Verschwenken;
- Fig. 19C: eine Seitenansicht des medizinischen Instruments bei weiterem Verschwenken; und
- Fig. 19D: eine Seitenansicht des medizinischen Instruments in einem verschwenkten Zustand.

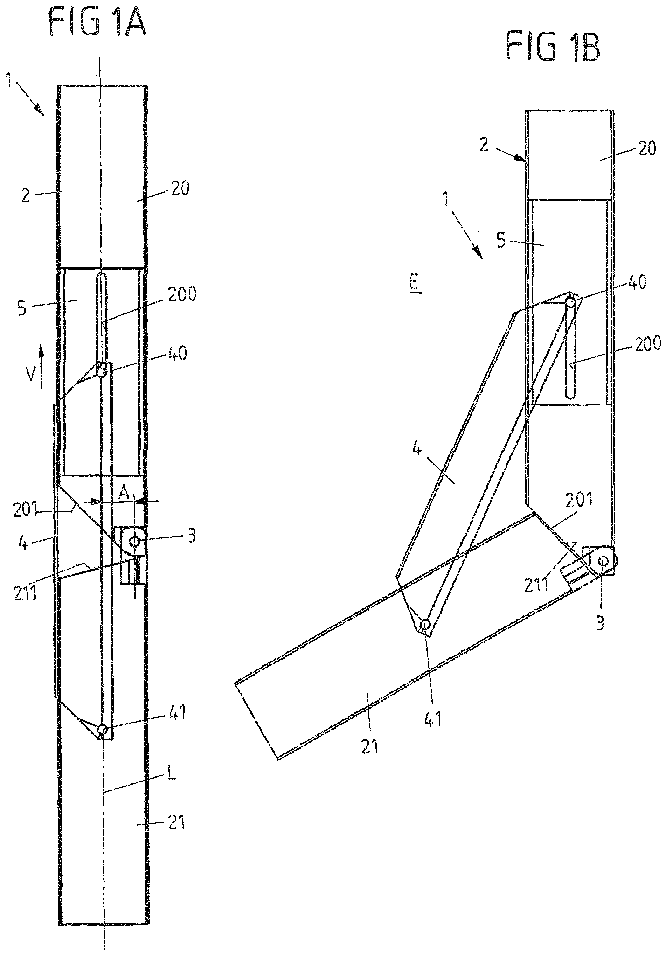

Fig. 1A und 1B zeigen Ansichten eines ersten Ausführungsbeispiels eines medizinischen Instruments 1, das einen aus zwei Schaftsegmenten 20, 21 gebildeten Schaft 2 aufweist, in dem beispielsweise ein Werkzeug wie ein Greifer, eine Zange oder ein Schneidwerkzeug hin zu einem Operationsort geführt werden kann oder an dessen distaler Spitze sich ein Aktuator befindet, so dass innerhalb des Schafts nur Kräfte und Momente zur Manipulation übertragen werden müssen.
Bei den dargestellten Ansichten stellt das obere, erste Schaftsegment 20 ein proximales Schaftsegment dar, das über ein Gelenk 3 mit einem unteren, distalen, zweiten Schaftsegment 21 verbunden ist. Mit dem distalen Schaftsegment 21 wird der Schaft 2 einem Operationsort zugeführt, derart, dass ein aus dem vom proximalen Schaftsegment 20 entfernten Ende des distalen Schaftsegments 21 austretendes Werkzeug an dem Operationsort operieren kann.

Die Schaftsegmente 20, 21 sind über das Gelenk 3 zueinander verschwenkbar. Zum Verschwenken der Schaftsegmente 20, 21 zueinander ist ein Verbindungselement 4 in Form eines Halbrohres vorgesehen, das über eine erste Gelenkverbindung 40 gelenkig mit einem im Inneren des Schaftsegments 20 geführten Verstellelement 5 und über eine zweite Gelenkverbindung 41 gelenkig mit dem Schaftsegment 21 gekoppelt ist. Das Verstellelement 5 kann entlang einer Verstellrichtung V, die entlang einer Längsachse L des proximalen, ersten Schaftsegments 20 gerichtet ist, im Inneren des Schaftsegments 20 verstellt werden, so dass die Schaftsegmente 20, 21 zueinander verschwenkt werden können.

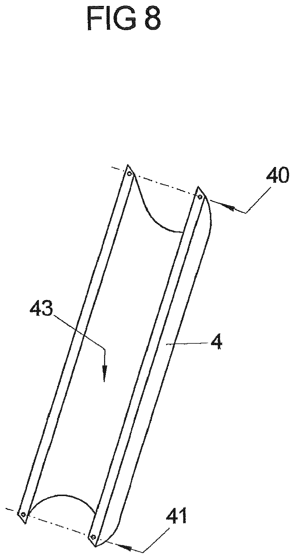

Das Verbindungselement 4 ist als Halbrohr ausgebildet, wie schematisch in Fig. 8 veranschaulicht. Das Verbindungselement 4 ist in sich gewölbt und hat die Form eines mittig entlang der Längsachse L geteilten, halbzylindrischen Rohrs, das in einem Längserstreckungszustand des Schafts 2 die Schaftsegmente 20, 21, wie in Fig. 1A dargestellt, abschnittsweise umgreift und mit einer inneren Anlagefläche 43 einen Anschlag für die Schaftsegmente 20, 21 in dem Längserstreckungszustand bereitstellt, so dass die Schaftsegmente 20, 21 nicht über den Längserstreckungszustand hinaus zueinander verschwenkt werden können.

Abweichend von der Erfindung kann das Verbindungselement 4 grundsätzlich auch eine von einem Halbrohr abweichende Form aufweisen und kann beispielsweise als stegartig oder plattenartig erstrecktes Element ausgebildet sein.

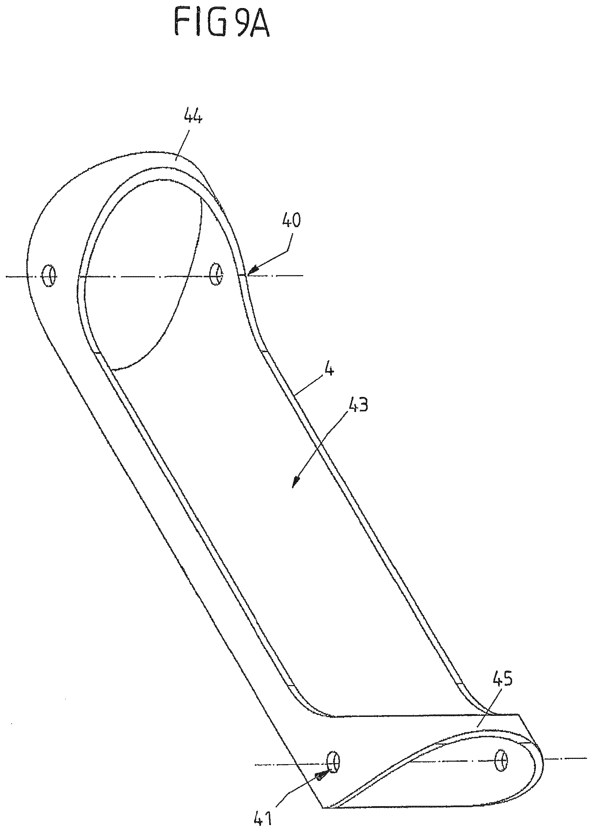

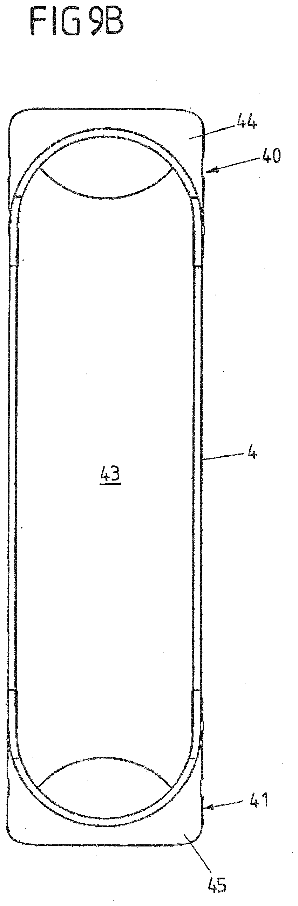

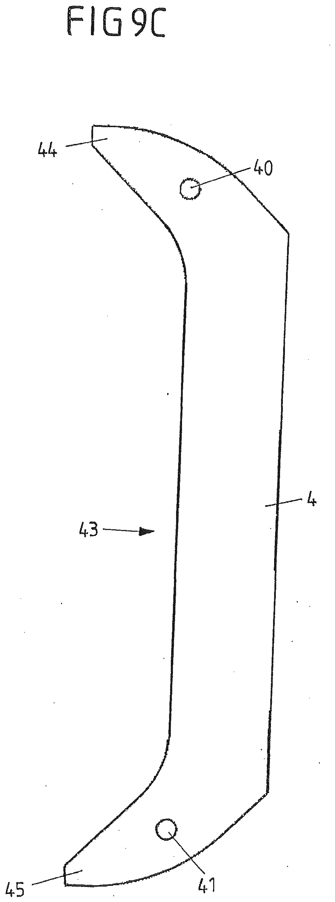

Ein modifiziertes Ausführungsbeispiel eines Verbindungselements 4 ist in Fig. 9A bis 9C dargestellt. Das Verbindungselement 4 weist eine grundsätzlich einem Halbrohr entsprechende Form auf, wobei an Enden 44, 45 des Verbindungselements 4 geschlossene Ringe ausgebildet sind, durch die hindurch das jeweils zugeordnete Schaftsegment 20, 21 greift. Das Verbindungselement 4 umschließt somit mit seinen Enden 44, 45 die Schaftsegmente 20, 21, was die Stabilität der Anordnung verbessern kann.

Wie aus Fig. 1A ersichtlich, liegen die Gelenkverbindungen 40, 41, über die das Verbindungselement 4 mit den Schaftsegmenten 20, 21 gekoppelt ist, auf einer Linie L, die - betrachtet in der Projektion auf eine Schwenkebene E, in der die Schaftsegmente 20, 21 um das Gelenk 3 zueinander verschwenkbar (siehe Fig. 1B) - der Längsachse der Schaftsegmente 20, 21 in dem Längserstreckungszustand des Schafts 2 entspricht. Von dieser Linie L ist das Gelenk 3 um einen Abstand A in Querrichtung beabstandet, wobei das Gelenk 3 in eine Richtung weg von dem Verbindungselement 4 von der Linie L versetzt ist.

Dies führt dazu, dass bei einem Verstellen des Verstellelements 5 aus dem Längserstreckungszustand heraus in die Verstellrichtung V das Gelenk 3 von dem Verbindungselement 4, wie aus Fig. 1B ersichtlich, entfernt wird, so dass das Verbindungselement 4 zusammen mit den Schaftsegmenten 20, 21 ein Dreieck aufspannt, dessen Dreiecksfläche sich mit dem Verschwenken der Schaftsegmente 20, 21 zueinander verändert.

Dadurch, dass das Verbindungselement 4 gelenkig an den Schaftsegmenten 20, 21 angreift, wird ein Dreigelenk geschaffen, bei dem das Verbindungselement 4 als abstützende Strebe in verschwenktem Zustand der Schaftsegmente 20, 21 wirkt, so dass die Schaftsegmente 20, 21 in verschwenktem Zustand in vorteilhafter Weise verstärkend zueinander abgestützt sind.

Wie aus Fig. 1A ersichtlich, weisen die Schaftsegmente 20, 21 an den einander zugewandten Enden jeweils eine Phase auf, die jeweils einen Anschlag 201, 211 bildet.

Diese Anschläge 201, 211 begrenzen den Schwenkweg der Schaftsegmente 20, 21 zueinander derart, dass in maximal verschwenktem Zustand, dargestellt in Fig. 1B, die Anschläge 201, 211 aneinander anliegen und somit ein weiteres Verschwenken der Schaftsegmente 20, 21 zueinander nicht möglich ist.

Das Verstellelement 5 ist im Inneren des proximalen Schaftsegments 20 geführt. Das Verbindungselement 4 ist über zugeordnete, durch zwei Zapfen ausgebildete Gelenkverbindung 40 mit dem Verstellelement 5 gekoppelt, wobei die die Gelenkverbindung 40 ausbildenden Zapfen Langlöcher 200 an diametral gegenüber liegenden Seiten des Schaftsegments 20 durchgreifen und darüber in Verbindung mit dem im Inneren des Schaftsegments 20 geführten Verstellelement 5 stehen.

Das Verstellelement 5 kann beispielsweise in motorisch angetriebener Weise verstellt werden, wobei das Verstellen motorisch angetrieben selbsttätig erfolgen oder in kraftunterstützter Weise bewirkt werden kann. In letzterem Fall wird eine manuelle Verstellbewegung des Verstellelements 5, bewirkt durch einen Nutzer, in motorischer Weise nach Art eines Servomotors kraftunterstützt, so dass ein Verstellen in leichtgängiger Weise bewirkt werden kann.

Dadurch, dass das Gelenk 3 beim Verschwenken der Schaftsegmente 20, 21 zueinander von dem Verbindungselement 4 entfernt wird, verstärkt sich die abstützende Wirkung des Verbindungselements 4 bei der Verschwenkbewegung aus dem Längserstreckungszustand heraus. Dies führt dazu, dass auf das Gelenk 3 wirkende Lastmomente in vorteilhafter Weise abgestützt werden und somit eine leichte, einfache Dimensionierung des Gelenks 3 möglich wird.

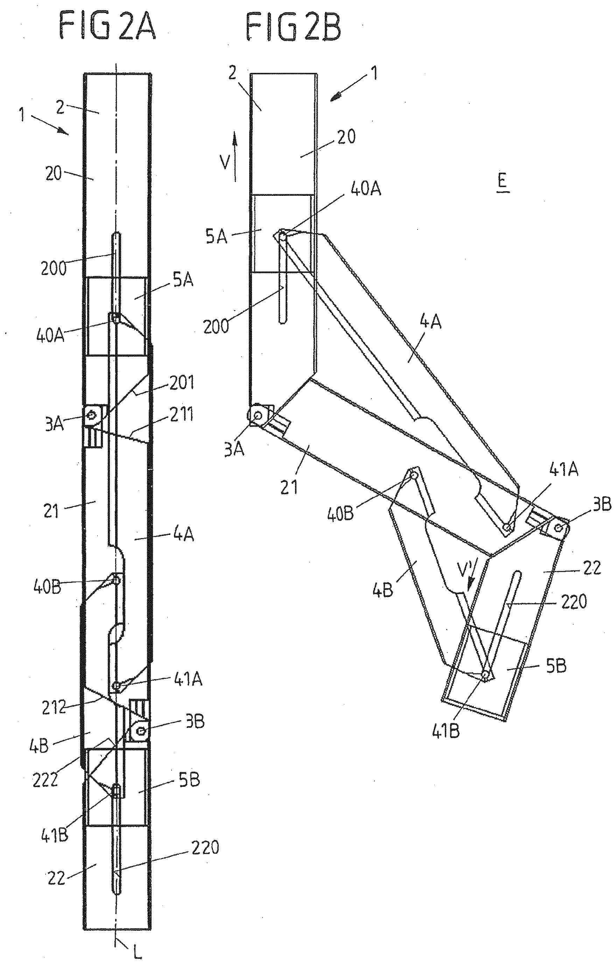

Bei einem in Fig. 2A, 2B dargestellten Ausführungsbeispiel eines medizinischen Instruments weist ein Schaft 2 drei Schaftsegmente 20, 21, 22 auf, die über Gelenke 3A, 3B gelenkig miteinander verbunden sind. Das medizinische Instrument 1 ist für den Zugang zu gegebenenfalls auch schwer zugänglichen Operationsorten geeignet und kann insbesondere auch für einen so genannten "Single Port"-Eingriff eingesetzt werden, bei dem verschiedene medizinische Instrumente 1 über einen einzigen Zugang einem Operationsort beispielsweise im Bauchraum eines Patienten zugeführt werden.

Bezugszeichen gleicher Funktion sollen im Weiteren, soweit zweckdienlich, mit gleichen Bezugszeichen versehen werden.

Bei dem Ausführungsbeispiel gemäß Fig. 2A und 2B sind zwei Verbindungselemente 4A, 4B vorgesehen, die jeweils über zwei Gelenkverbindungen 40A, 41A bzw. 40B, 41B gelenkig mit den zugeordneten Schaftsegmenten 20, 21, 22 verbunden sind. Das erste Verbindungselement 4A ist hierbei über eine erste Gelenkverbindung 40A gelenkig an einem im Inneren des proximalen Schaftsegments 20 geführten Verstellelement 5A angelenkt und über eine zweite Gelenkverbindung 41A mit dem zweiten Schaftsegment 21 gekoppelt. Das zweite Verbindungselement 4B ist über eine dritte Gelenkverbindung 40B mit dem zweiten Schaftsegment 21 und über eine vierte Gelenkverbindung 41B mit einem im Inneren des dritten, distalen Schaftsegments 22 geführten Verstellelements 5B gekoppelt. Die Verbindungselemente 4A, 4B bilden jeweils für sich Halbrohre aus, wobei die Verbindungselemente 4A, 4B in unterschiedliche Richtungen geöffnet sind und in dem Längserstreckungszustand (siehe Fig. 2A) an diametral gegenüberliegenden Seiten an den jeweils zugeordneten Schaftsegmenten 20, 21, 22 anliegen.

Die Verbindungselemente 4A, 4B können bei dem dargestellten Ausführungsbeispiel durch Verstellen der Verstellelemente 5A, 5B in unabhängiger Weise zueinander verstellt werden. Durch Verstellen des ersten Verstellelements 5A können hierbei das erste Schaftsegment 20 und das zweite Schaftsegment 21 zueinander verschwenkt werden. Durch Verstellen des zweiten Verstellelements 5B hingegen können das zweite Schaftsegment 21 und das dritte Schaftsegment 22 zueinander verschwenkt werden, wobei das Verschwenken jeweils aus dem in Fig. 2A dargestellten Längserstreckungszustand heraus in unterschiedliche Richtungen, aber in der gleichen Schwenkebene E erfolgt, und der maximale Schwenkwinkel jeweils durch Anschläge 201, 211 bzw. 212, 222 an Phasen der Schaftsegmente 20, 21, 22 bestimmt ist.

Bei dem dargestellten Ausführungsbeispiel sind die Schaftsegmente 20, 21, 22 grundsätzlich unabhängig zueinander verschwenkbar. Vorgesehen sein kann aber, dass die Verstellbewegung der Verstellelemente 5A, 5B elektronisch miteinander gekoppelt ist, so dass ein Verschwenken des ersten und zweiten Schaftsegments 20, 21 zueinander immer mit einem Verschwenken des zweiten und dritten Schaftsegments 21, 22 zueinander einhergeht und die Verschwenkbewegung somit in gekoppelter Weise erfolgt.

Das zweite Verstellelement 5B wird zum Verschwenken des dritten Schaftsegments 22 relativ zu dem zweiten Schaftsegment 21 in eine Verstellrichtung V' verstellt, die der Verstellrichtung V des ersten Verstellelements 5A entgegengesetzt gerichtet ist. Dies ist dadurch bedingt, dass das Verstellelement 5B an dem dritten Schaftsegment 22 geführt ist und zum Verschwenken des dritten Schaftsegments 22 relativ zu dem zweiten Schaftsegment 21 das Verstellelement 5B vom Gelenk 3B zu entfernen ist.

Die Verstellelemente 5A, 5B sind jeweils im Inneren der zugeordneten Schaftsegmente 20, 22 geführt, wobei die zugeordneten Verbindungselemente 4A, 4B jeweils über Langlöcher 200, 220 über zapfenförmige Gelenkverbindungen 40A, 41B mit dem zugeordneten Verstellelement 5A, 5B gekoppelt sind.

Wie aus Fig. 2A ersichtlich, liegen in dem Längserstreckungszustand des Schafts 2 die Gelenkverbindungen 40A, 40B, 41A, 41B auf einer Linie L, die - betrachtet in der Projektion auf die Schwenkebene E - der Längsachse L des Schafts 2 in dem Längserstreckungszustand entspricht. Die Gelenke 3A, 3B, über die die einzelnen Schaftsegmente 20, 21, 22 gelenkig miteinander verbunden sind, sind zu unterschiedlichen Richtungen von dieser Linie L versetzt, wobei jedes Gelenk 3A, 3B zu einer von dem jeweils zugeordneten Verbindungselement 4A, 4B weg weisenden Richtung von der Linie L beabstandet ist. Dies ermöglicht ein einfaches Verschwenken der Schaftsegmente 20, 21, 22 zueinander, ohne dass Totpunkte bei der Schwenkbewegung gebildet werden.

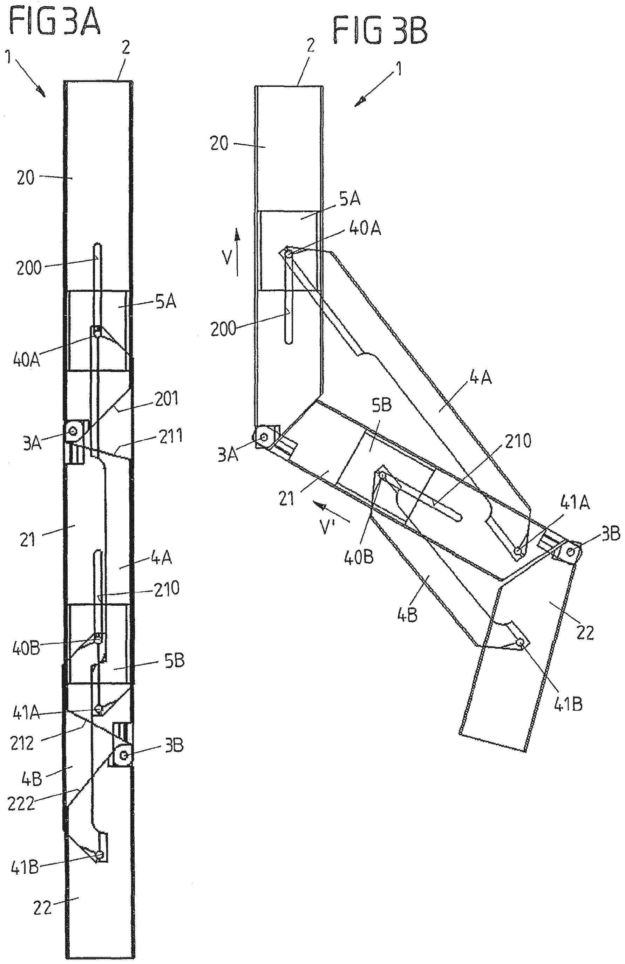

Bei einem modifizierten, in Fig. 3A, 3B dargestellten Ausführungsbeispiel ist das zweite Verstellelement 5B verstellbar an dem zweiten Schaftsegment 21 geführt und zum Verschwenken des dritten Schaftsegments 22 relativ zu dem zweiten Schaftsegment 21 in eine der Verstellrichtung V des ersten Verstellelementes 5A gleichgerichteten Verstellrichtung V' zu verstellen. Es ergibt sich eine geringfügig andere Kinematik bei ansonsten gleicher Funktionsweise des medizinischen Instruments 1.

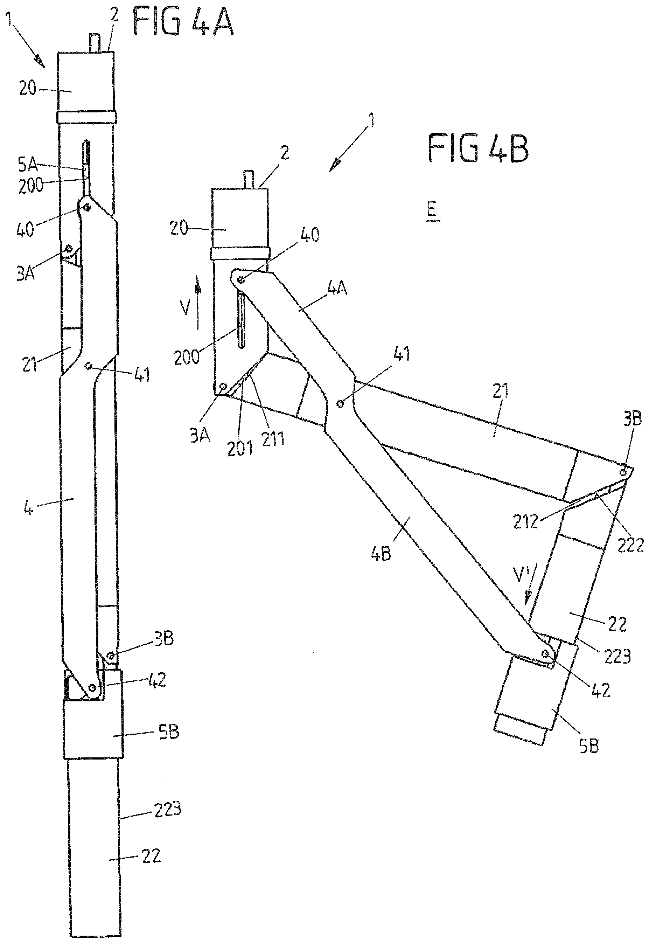

Bei einem in Fig. 4A, 4B dargestellten Ausführungsbeispiel sind zwei Verbindungselemente 4A, 4B vorgesehen, die starr miteinander gekoppelt sind, so dass sich eine mechanisch gekoppelte Verschwenkbewegung dreier Schaftsegmente 20, 21, 22 zueinander ergibt.

Ein erstes Verbindungselement 4A ist in diesem Fall gelenkig über eine erste Gelenkverbindung 40 mit einem ersten Verstellelement 5A an dem ersten Schaftsegment 20 und gelenkig über eine zweite Gelenkverbindung 41 mit dem zweiten Schaftsegment 21 gekoppelt. Das zweite Verbindungselement 4B, das einstückig mit dem ersten Verbindungselement 4A ausgebildet ist, ist über die zweite Gelenkverbindung 41 mit dem zweiten Schaftsegment 21 und gelenkig mit einem zweiten, als Hülse ausgebildeten und an einem Schaftabschnitt 223 des zweiten Schaftsegments 22 geführten zweiten Verstellelement 5B über die Gelenkverbindung 42 verbunden.

Bei dem medizinischen Instrument 1 gemäß Fig. 4A, 4B wird das Verstellelement 5B bei einer angetriebenen Bewegung des ersten Verstellelements 5A in passiver Weise mitbewegt, ohne dass das zweite Verstellelement 5B selbst anzutreiben ist. Wird das erste Verstellelement 5A in die zugeordnete Verstellrichtung V bewegt, so wird das zweite Schaftsegment 21 relativ zu dem ersten Schaftsegment 20 verschwenkt, und gleichzeitig gleitet das zweite Verstellelement 5B in eine entgegengesetzte, zugeordnete Verstellrichtung V' an dem Schaftabschnitt 223 des dritten Schaftsegments 22, so dass auch das dritte Schaftsegment 22 relativ zu dem zweiten Schaftsegment 21 bewegt wird. Die Schwenkbewegung erfolgt in einer Schwenkebene E, wobei die Schwenkbewegung des ersten und zweiten Schaftsegments 20, 21 zueinander entgegengesetzt ist zu der Schwenkbewegung des zweiten und dritten Schaftsegments 21, 22 zueinander.

Die Verbindungselemente 4A, 4B sind wiederum jeweils als Halbrohr ausgebildet, wobei die Verbindungselemente 4A, 4B zu unterschiedlichen Seiten geöffnet sind und im Längserstreckungszustand (siehe Fig. 4A) an diametral gegenüberliegenden Seiten an den zugeordneten Schaftsegmenten 20, 21, 22 anliegen. Die Verbindungselemente 4A, 4B bilden auf diese Weise jeweils einen Anschlag für die zugeordneten Schaftsegmente 20, 21, 22, so dass die Schaftsegmente 20, 21, 22 nicht über den Längserstreckungszustand hinaus zueinander verschwenkt werden können.

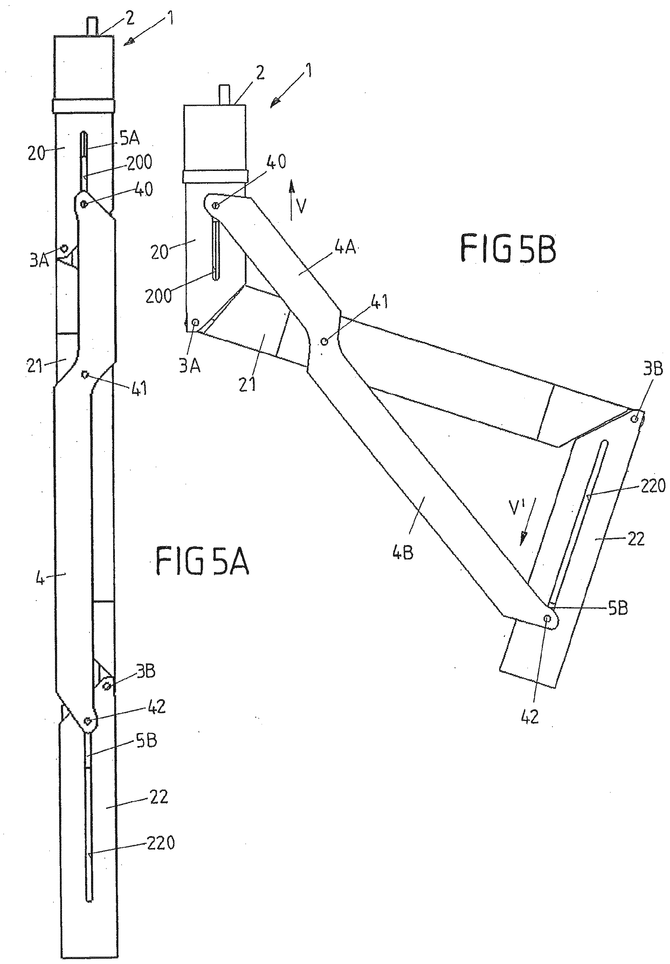

Bei dem Ausführungsbeispiel gemäß Fig. 4A, 4B ist das zweite Verstellelement 5B als an dem Schaftabschnitt 223 des dritten Schaftsegments 22 außen geführte Hülse ausgebildet. Selbstverständlich ist auch möglich, wie bei dem Ausführungsbeispiel gemäß Fig. 5A, 5B verwirklicht, das zweite Verstellelement 5B als innen im dritten Schaftsegment 22 geführtes Verstellelement 5B auszubilden, das über Langlöcher 220 (die sich diametral an dem zylindrischen Schaftsegment 22 gegenüberliegen) mit der durch Zapfen gebildeten Gelenkverbindung 42 und darüber mit dem zweiten Verbindungselement 4B in Wirkverbindung steht. Ansonsten ist das Ausführungsbeispiel gemäß Fig. 5A, 5B funktionsgleich dem Ausführungsbeispiel gemäß Fig. 4A, 4B.

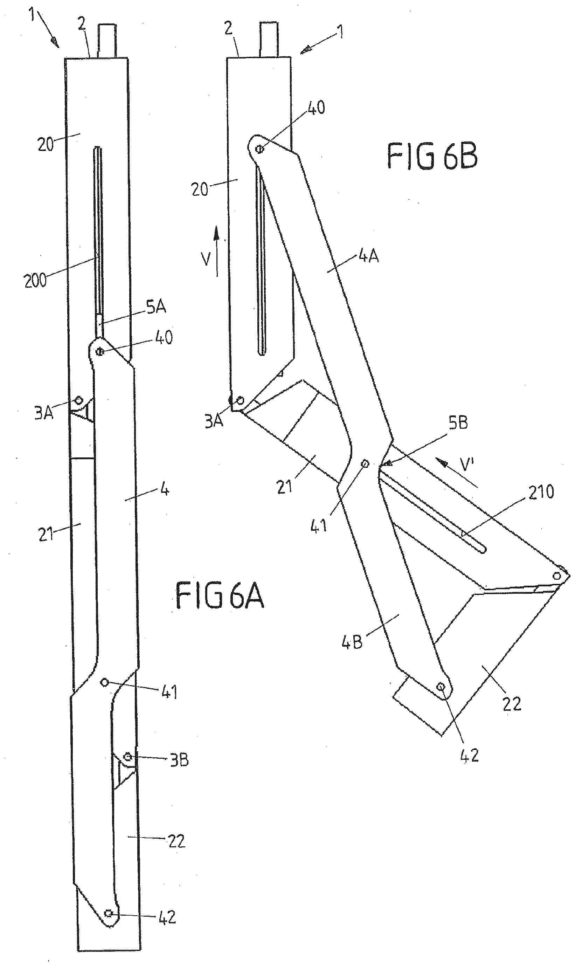

Bei dem Ausführungsbeispiel gemäß Fig. 6A, 6B ist, im Unterschied zum Ausführungsbeispiel gemäß Fig. 5A, 5B, das zweite Verstellelement 5B verstellbar an dem zweiten, mittleren Schaftsegment 21 geführt, läuft im Inneren des rohrförmigen Schaftsegments 21 und steht über Langlöcher 210 mit der zweiten Gelenkverbindung 41, die den Verbindungselementen 4A, 4B gemeinsam zugeordnet ist, in Wirkverbindung.

Dadurch, dass das zweite Verstellelement 5B an dem zweiten Schaftsegment 21 entlang einer der Verstellrichtung V des ersten Verstellelements 5A gleichgerichteten Verstellrichtung V' verschiebbar geführt ist, ergibt sich eine andere Kinematik bei einem Verschwenken der Schaftsegmente 20, 21, 22 zueinander. Dadurch, dass das zweite Verbindungselement 4B über die Gelenkverbindung 42 ortsfest an dem dritten Schaftsegment 22 angelenkt ist, kann das dritte Schaftsegment 22 kürzer ausgebildet werden, was in Anbetracht des an einem Operationsort zur Verfügung stehenden begrenzten Raums vorteilhaft sein kann.

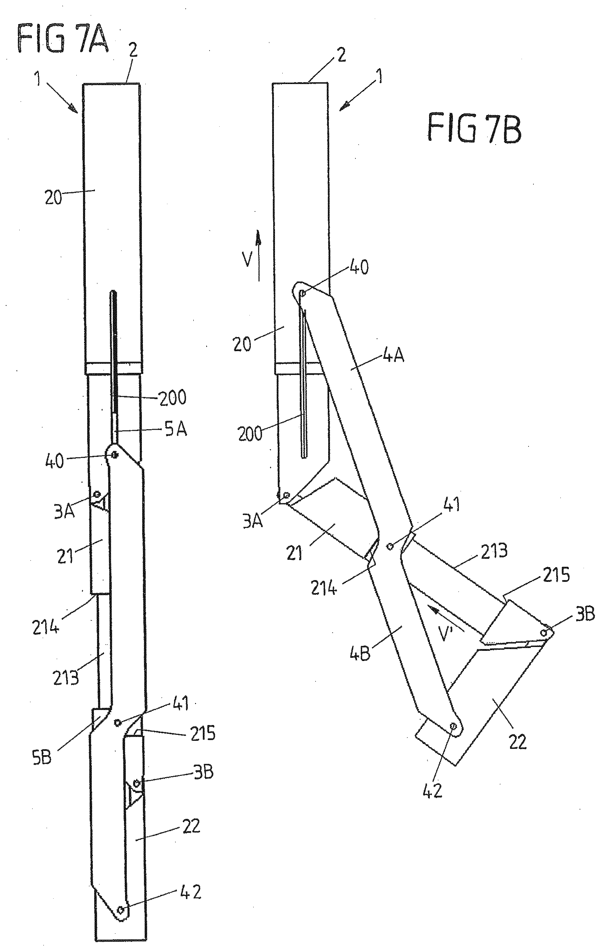

Wie in Fig. 7A, 7B dargestellt, kann das zweite Verbindungselement 5B auch als Hülse an einem Schaftabschnitt 213 des zweiten Schaftsegments 21 geführt und zwischen Anschlägen 214, 215, die die Bewegungsbahn begrenzen, verstellbar sein. Ansonsten ist das Ausführungsbeispiel gemäß Fig. 7A, 7B funktionsgleich dem Ausführungsbeispiel gemäß Fig. 6A, 6B.

Durch Vorgabe der Längen der Verbindungselemente 4A, 4B und durch Auswahl der Anlenkungspunkte der Gelenkverbindungen 40, 41, 42 bzw. 40A, 41A, 40B, 41B kann die Kinematik bei einem Verschwenken der Schaftsegmente 20, 21, 22 vorgegeben werden. Zudem können die einzelnen Schaftsegmente 20, 21, 22 mit geeigneten Längen gewählt werden, so dass sich eine gewünschte Auslenkung der Schaftsegmente 20, 21, 22 ergibt.

Bei der dreisegmentigen Ausführung des Schafts 2 wird das zweite Schaftsegment 21 zunächst nach außen und von diesem ausgehend das dritte Schaftsegment 22 dann zurück nach innen verschwenkt. Dies erlaubt insbesondere den Einsatz des medizinischen Instruments 1 im Rahmen eines "Single Port"-Zugangs, um verschiedenen medizinische Instrumenten 1 einen Zugang hin zu einem Operationsort zu ermöglichen, ohne dass die medizinischen Instrumente sich dabei in ihrer Bewegung gegenseitig stören.

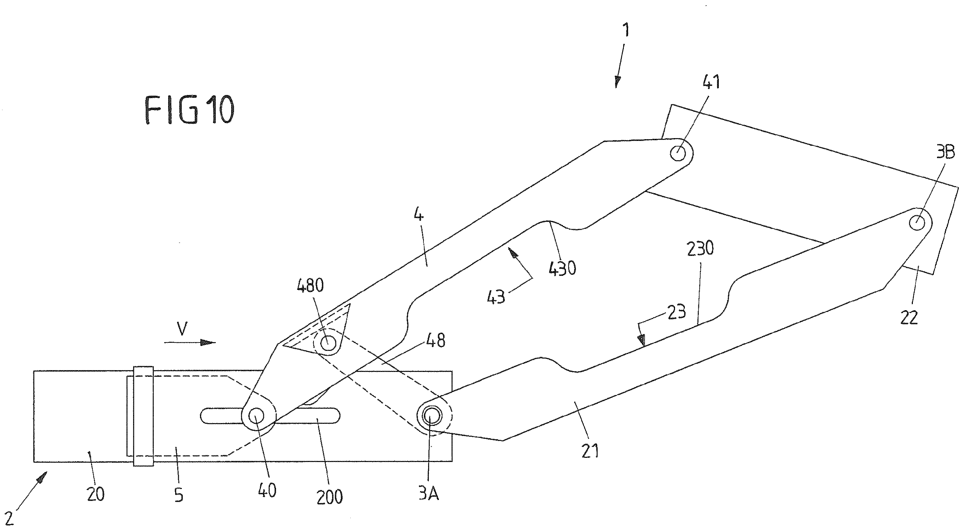

Bei einem weiteren, in Fig. 10 bis 12A-12C dargestellten Ausführungsbeispiel eines medizinischen Instruments 1 weist ein Schaft 2 drei Schaftsegmente 20, 21, 22 auf. Das zweite Schaftsegment 21, das nach Art eines Halbrohrs ausgebildet ist und somit halbseitig offen ist, ist über eine Gelenkverbindung 3A mit dem ersten Schaftsegment 20 und über eine Gelenkverbindung 3B mit dem dritten Schaftsegment 22 verbunden. Zusätzlich ist das erste Schaftsegment 20 über ein Verbindungselement 4 mit dem dritten Schaftsegment 22 gekoppelt, wobei das Verbindungselement 4 über eine Gelenkverbindung 41 mit dem dritten Schaftsegment 22 und über eine Gelenkverbindung 40 mit einem längsverschieblich entlang einer Verstellrichtung V an dem ersten Schaftsegment 20 geführten Verstellelement 5 gekoppelt ist.

Das erste Schaftsegment 20, das zweite Schaftsegment 21, das dritte Schaftsegment 22 und das Verbindungselement 4 bilden zusammen ein Viergelenk aus. Um hierbei bei einem Verstellen des Verstellelements 5 eine Trajektorie, entlang derer das dritte Schaftsegment 22 zu bewegen ist, festzulegen, ist das Verbindungselement 4 zusätzlich über ein Hebelelement 48 mit dem ersten Schaftsegment 20 gekoppelt. Das Hebelelement 48 ist über ein Gelenk 480 dabei mit dem Verbindungselement 4 gelenkig verbunden und zudem über die Gelenkverbindung 3A, an der auch das zweite Schaftsegment 21 mit dem ersten Schaftsegment 20 gekoppelt ist, an dem ersten Schaftsegment 20 angelenkt.

Das Verbindungselement 4 ist (beispielsweise näherungsweise spiegelbildlich zum zweiten Schaftsegment 21) nach Art eines hin zum zweiten Schaftsegment 21 offenen Halbrohrs ausgebildet und umgreift einerseits das erste Schaftsegment 20 und andererseits das dritte Schaftsegment 22. Die Gelenkverbindung 40 ist hierbei durch einen Zapfen hergestellt, der eine gelenkige Verbindung mit dem Verstellelement 5 schafft und ein Langloch 200 an dem ersten Schaftsegment 20 durchgreift, so dass über den die Gelenkverbindung 40 ausbildenden Zapfen das Verbindungselement 4 auch entlang der Verstellrichtung V längsverschieblich an dem ersten Schaftsegment 20 geführt ist (das erste Schaftsegment 20 weist beidseitig angeordnete, diametral gegenüberliegende Langlöcher 200 auf, die jeweils von einem Zapfen zur gelenkigen Anbindung des Verbindungselements 4 beidseitig an das Verstellelement 5 durchgriffen werden, von denen in Fig. 10 aber nur ein Langloch 200 dargestellt ist).

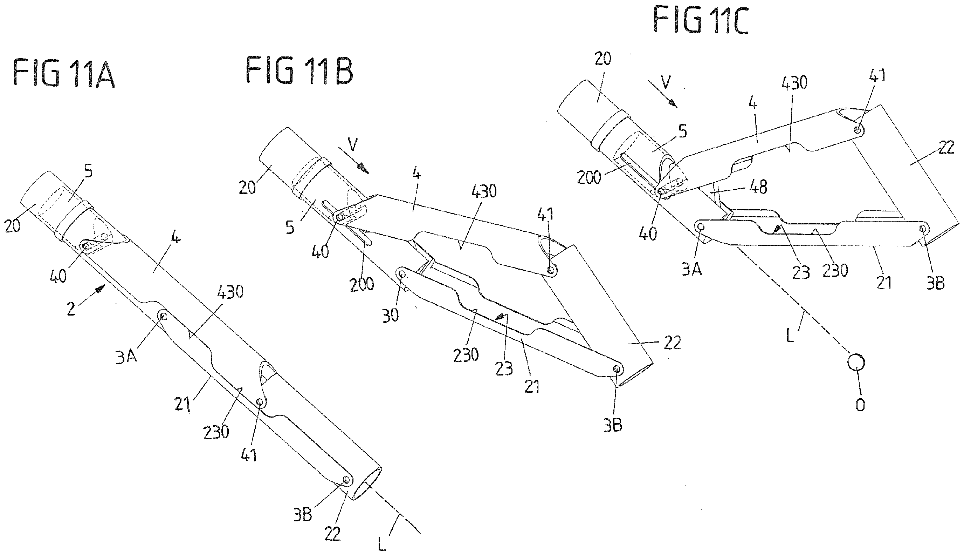

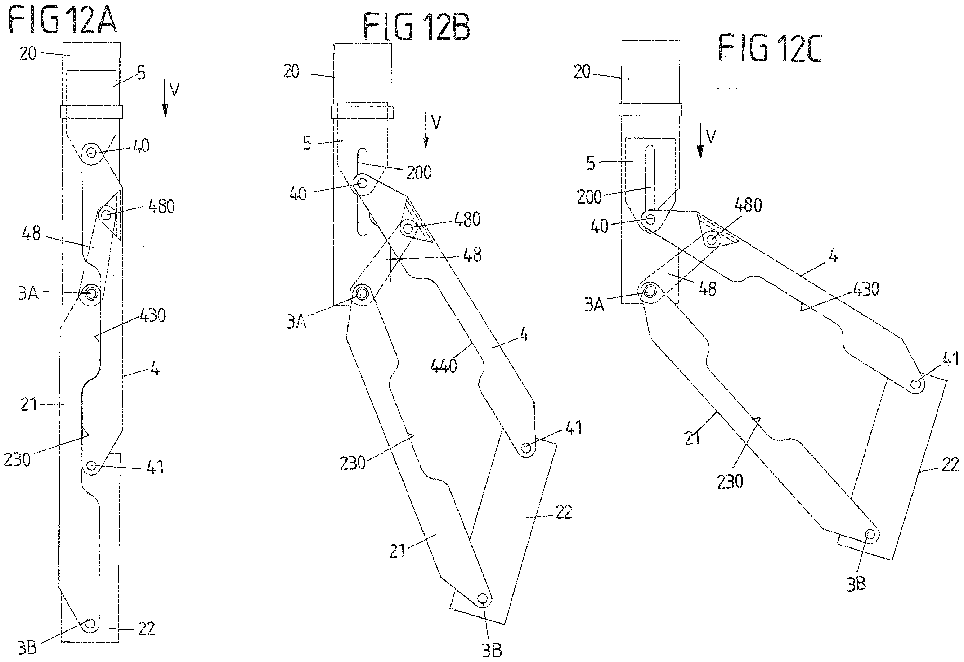

In einem Längserstreckungszustand, dargestellt in Fig. 11A und 12A, fluchten die Schaftsegmente 20, 21, 22 und auch das Verbindungselement 4 miteinander. Das halbrohrförmige Verbindungselement 4 und das halbrohrförmige zweite Schaftsegment 21 sind hierbei einander angenähert, wobei das zweite Schaftsegment 21 mit einem dem Gelenkteil A zugeordneten Ende in einer Aussparung 430 in den Wandungen des Verbindungselements 4 einliegt und das Verbindungselement 4 mit einem der Gelenkverbindung 41 zugeordneten Ende in einer entsprechenden Aussparung 230 in den Wandungen des zweiten Schaftsegments 21 angeordnet ist. Es ergibt sich durch Anlage des Verbindungselements 4 und des zweiten Schaftsegments 21 aneinander eine im Wesentlichen geschlossene Hülle, in der zumindest abschnittsweise das erste Schaftsegment 20 und das dritte Schaftsegment 22 aufgenommen sind.

Für ein Ausstellen des dritten Schaftsegments 22 wird das Verstellelement 5 in die Verstellrichtung V innerhalb des ersten Schaftsegments 20 verstellt. Weil das Gelenk 480, über das das Hebelelement 48 mit dem Verbindungselement 4 gekoppelt ist, quer zur Verstellrichtung zu einer die Gelenke 40, 3A, 41 und 3B verbindenden, gedachten Linie versetzt ist, wird durch die Verschiebebewegung des Verstellelements 5 das Verbindungselement 4 ausgeschwenkt, wie dies in Fig. 11B und 12B dargestellt ist, wobei das zweite Schaftsegment 21 und das dritte Schaftsegment dem Verbindungselement 4 folgen und ausgeschwenkt werden.

In einem verschwenkten Zustand, dargestellt in Fig. 11C und 12C, ist das dritte Schaftsegment 22 quer zur Längsachse des ersten Schaftsegments 20 von dem ersten Schaftsegment 20 entfernt und dabei derart relativ zu dem ersten Schaftsegment 20 verschwenkt, dass ein von dem Gelenk 41 abliegendes Ende des dritten Schaftsegments 22 nach innen hin zu einer Längsachse L weist, entlang derer sich das erste Schaftsegment 20 erstreckt. Über das dritte Schaftsegment 22 kann somit ein Werkzeug hin zu einem Operationsort O (siehe Fig. 11C) geführt werden, um an diesem Operationsort O einen gewünschten Eingriff durchzuführen.

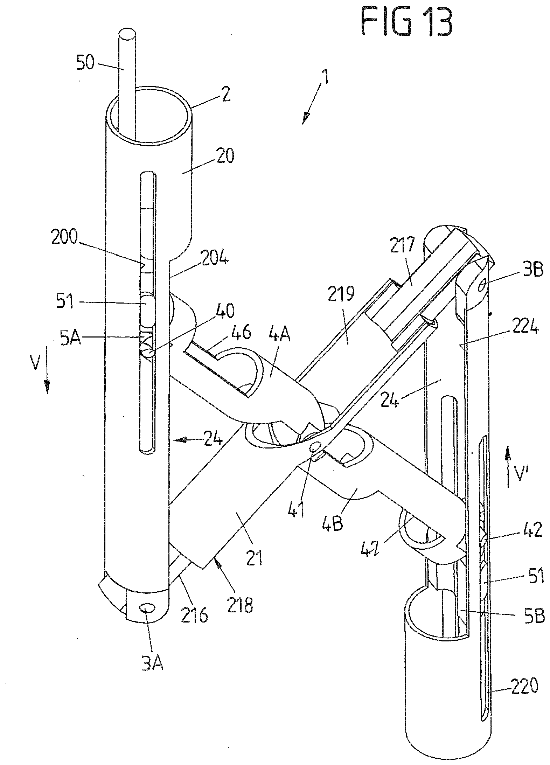

Ein weiteres Ausführungsbeispiel eines medizinischen Instruments 1 mit einem Schaft 2, der drei Schaftsegmente 20, 21, 22 aufweist, ist in Fig. 13 bis 17 dargestellt.

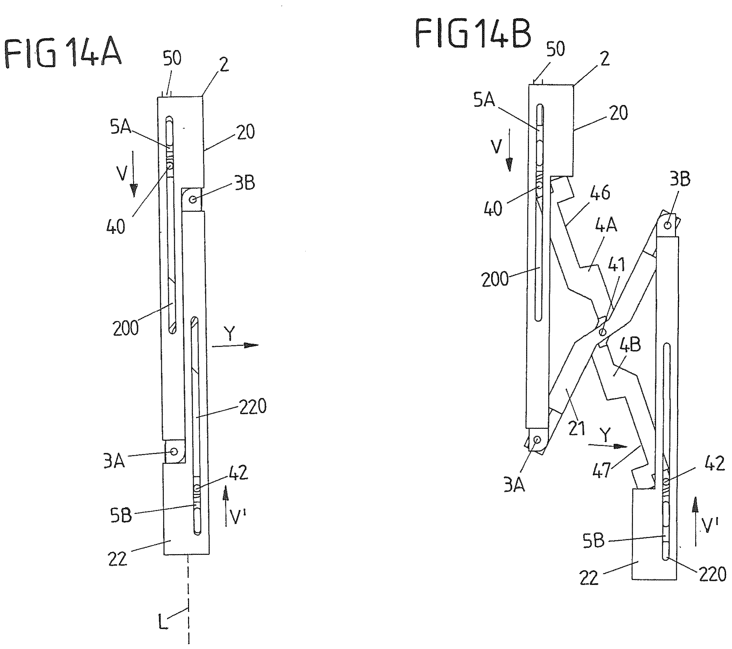

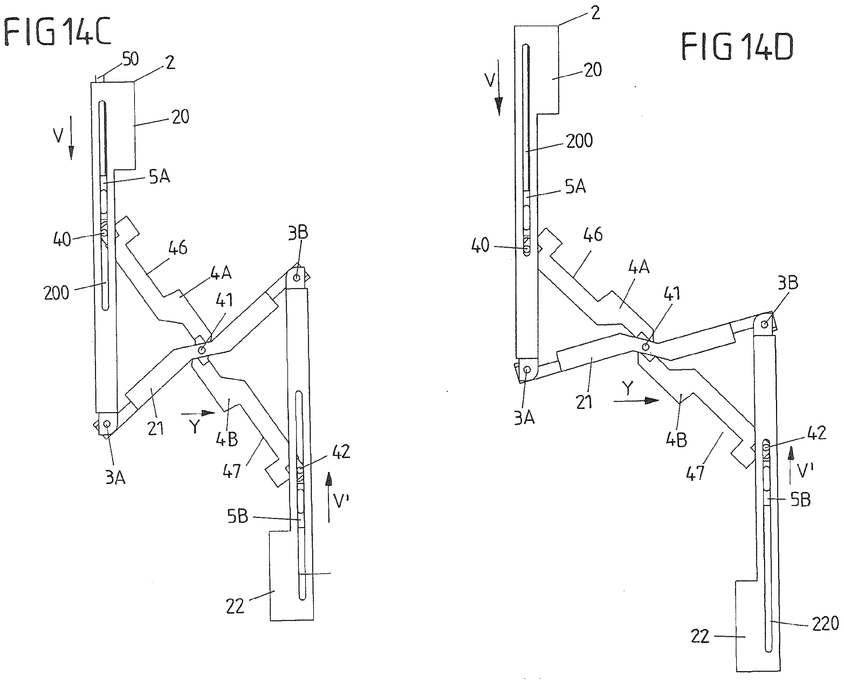

Das Ausführungsbeispiel gemäß Fig. 13 bis 17 unterscheidet sich beispielsweise von dem Ausführungsbeispiel gemäß Fig. 5A, 5B dadurch, dass in einem Längserstreckungszustand, dargestellt in Fig. 14A, das zweite Schaftsegment 21 und zwei einstückig ausgebildete, starr miteinander verbundene Verbindungselemente 4A, 4B innerhalb des ersten Schaftsegments 20 und des dritten Schaftsegments 22 aufgenommen und somit im Längserstreckungszustand eingefasst sind. Das erste Schaftsegment 20 und das dritte Schaftsegment 22 sind hierzu jeweils abschnittsweise nach Art eines Halbrohrs geformt, sind somit einseitig offen und weisen jeweils in ihrer Umfangskontur eine Aussparung 204, 224 auf. Das erste Schaftsegment 20 und das dritte Schaftsegment 22 bilden auf diese Weise jeweils eine halbrunde Aufnahme 24 zum Aufnehmen des zweiten Schaftsegments 21 und der Verbindungselemente 4A, 4B aus.

Bei dem Ausführungsbeispiel ist das zweite Schaftsegment 21 über ein Gelenk 3A mit dem ersten Schaftsegment 20 und über ein Gelenk 3B mit dem dritten Schaftsegment 22 gelenkig, aber ortsfest gekoppelt. Die Herstellung der Verbindung des zweiten Schaftsegments 21 mit dem ersten Schaftsegment 20 bzw. dem dritten Schaftsegment 22 erfolgt hierbei über je ein Verbindungsstück 216, 217, das an je einem halbrohrförmigen Abschnitt des zweiten Schaftsegments 21 gehalten ist.

Zwischen dem ersten Schaftsegment 20 und dem zweiten Schaftsegment 21 erstreckt sich ein erstes Verbindungselement 4A, das über eine Gelenkverbindung 41 mit dem zweiten Schaftsegment 21 und über eine Gelenkverbindung 40 in Form eines Zapfens mit einem längsverschieblich an dem ersten Schaftsegment 20 geführten Verstellelement 5A verbunden ist. Das erste Verbindungselement 4A ist starr und einstückig mit einem zweiten Verbindungselement 4B ausgebildet, das sich zwischen dem zweiten Schaftsegment 21 und dem dritten Schaftsegment 22 erstreckt und über die Gelenkverbindung 41 mit dem zweiten Schaftsegment 21 und über eine Gelenkverbindung 42 mit einem an dem dritten Schaftsegment 22 längs verschieblich geführten Verstellelement 5B gelenkig gekoppelt ist.

Die Verstellelemente 5A, 5B sind jeweils über stegförmig vorstehende Führungsabschnitte 51 an Langlöchern 200, 220 des zugeordneten Schaftsegments 20, 22 geführt, wobei jedes Schaftsegment 20, 22 zwei Langlöcher 200, 220 aufweist und entsprechend über beidseitige Führungsabschnitte 51 eine beidseitige Führung des jeweiligen Verstellelements 5A, 5B am zugeordneten Schaftsegment 20, 22 bereitgestellt wird.

In einem Längserstreckungszustand, dargestellt in Fig. 14A, erstrecken sich die Schaftsegmente 20, 21, 22 sowie auch die Verbindungselemente 4A, 4B entlang einer gemeinsamen Längsachse L, so dass die einzelnen Segmente kollinear zueinander angeordnet sind. Das zweite Schaftsegment 21 ist hierbei hin zum ersten Schaftsegment 20 verschwenkt und beschreibt (bezogen auf die Längsachsen) einen Winkel von 0°C zum ersten Schaftsegment 20. Entsprechend weist das zweite Schaftsegment 21 einen Winkel von 0°C zum dritten Schaftsegment 22 auf. Das erste Schaftsegment 20 und das dritte Schaftsegment 22 liegen im Längserstreckungszustand im Bereich ihrer Aussparungen 204, 224 aneinander an und bilden somit eine Hülle für das zweite Schaftsegment 21 und die Verbindungselemente 4A, 4B, die somit zwischen dem ersten Schaftsegment 20 und dem dritten Schaftsegment 22 aufgenommen sind. Die Verbindungselemente 4A, 4B sind hierbei innerhalb des zweiten Schaftsegments 21 angeordnet und liegen insbesondere in Aufnahmeöffnungen 218, 219 des zweiten Schaftsegments 21 ein, wobei die Verbindungsstücke 216, 217 des zweiten Schaftsegments 21 in Aussparungen 46, 47 der Verbindungselemente 4A, 4B zu liegen kommen.

Wird durch Verstellen des Verstellelements 5A des ersten Schaftsegments 20 über eine Betätigungsstange 50 eine Verstellkraft in die Verbindungselemente 4A, 4B eingeleitet und wird dadurch das die Gelenkverbindung 40 tragende Ende des ersten Verbindungselements 4A in eine Verstellrichtung V relativ zum ersten Schaftsegment 20 verstellt, so wird das zweite Schaftsegment 21 und darüber auch das dritte Schaftsegment 22 ausgeschwenkt. Weil die Verbindungselemente 4A, 4B gleich lang sind und auch die Gelenkverbindung 41 mittig am zweiten Schaftsegment 21 angeordnet ist, wird hierbei das dritte Schaftsegment 22 in eine Querrichtung Y quer zur Längsachse L des ersten Schaftsegments 20 parallel verschoben, wie dies in Fig. 14B bis 14D dargestellt ist. In einer ausgestellten Stellung, dargestellt in Fig. 14D, ist das dritte Schaftsegment 22 dann zum ersten Schaftsegment 20 quer entfernt, hat aber seine parallele Lage zum ersten Schaftsegment 20 beibehalten.

Anzumerken ist hierzu, dass die Parallelverschiebung nur einen Sonderfall aller möglichen Bewegungen darstellt und grundsätzlich auch eine andere Bewegung möglich ist.

Durch Verstellen des ersten Verstellelements 5A am ersten Schaftsegment 20 wird auch das zweite Verstellelement 5B am dritten Schaftsegment 22 verstellt. Das zweite Verstellelement 5B wird hierbei selbst nicht angetrieben, sondern lediglich bei Verstellung des ersten Verstellelements 5A passiv mitbewegt, wobei das zweite Verstellelement 5B sich in eine entgegengesetzte Verstellrichtung V' relativ zum ersten Verstellelement 5A bewegt.

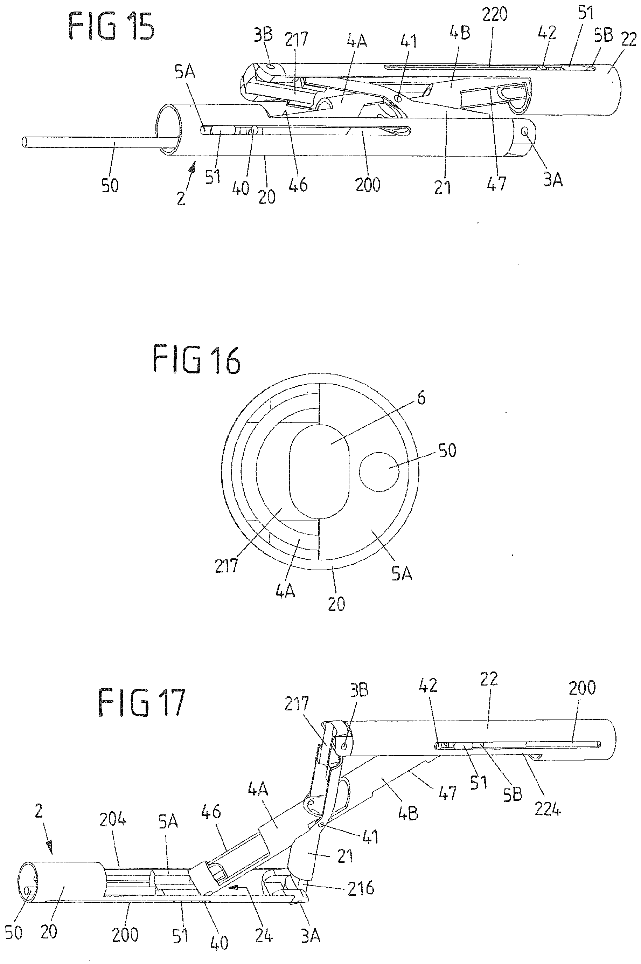

Aus Fig. 16 ist ersichtlich, wie das zweite Schaftsegment 21 mit seinem Verbindungsstück 217 sowie die Verbindungselemente 4A, 4B im Längserstreckungszustand in dem ersten Schaftsegment 20 und dem dritten Schaftsegment 22 einliegen. Die Verbindungsstücke 216, 217 des zweiten Schaftsegments 21 sowie die Verstellelemente 5A, 5B sind hierbei jeweils an einer radial nach innen weisenden Innenfläche derart geformt, dass sich eine Öffnung 6 ausbildet, durch die Übertragungselemente für Kräfte und Momente zu einem distal am dritten Schaftsegment 22 angeordneten Werkzeug geführt werden können. Solche Übertragungselemente können beispielsweise entlang der Verbindungselemente 4A, 4B verlegt werden, um vom ersten Schaftsegment 20 hin zum dritten Schaftsegment 22 geführt zu werden.

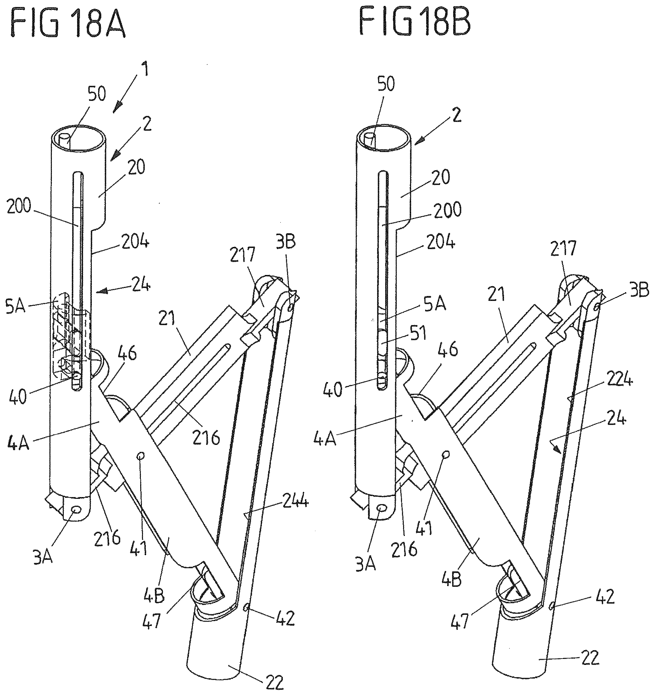

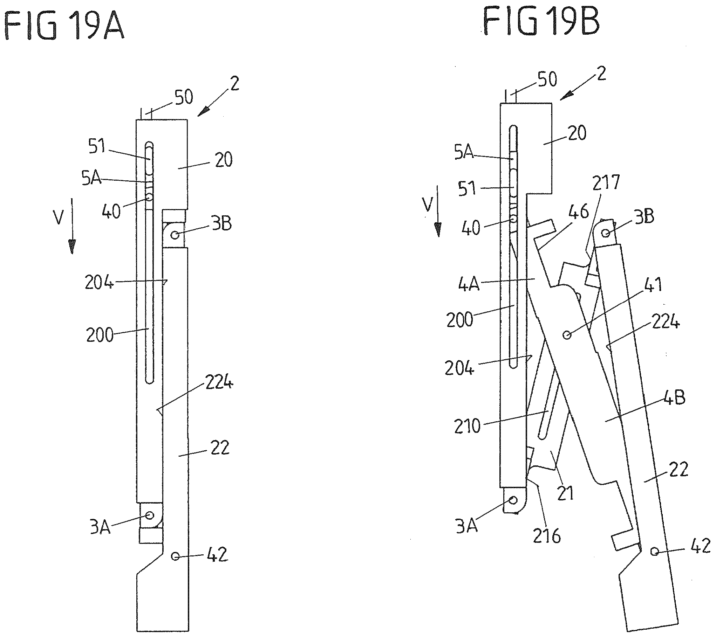

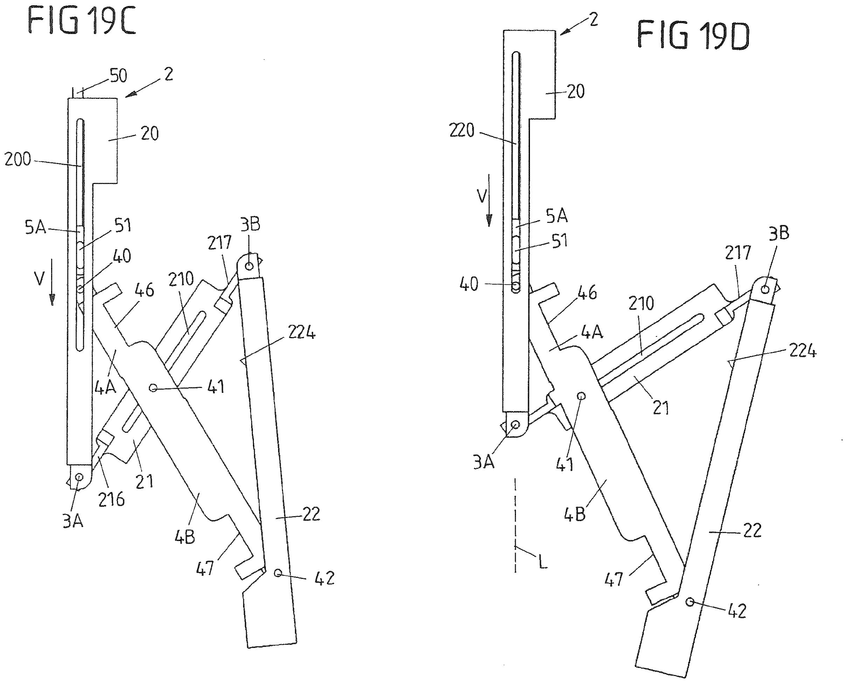

Bei einem weiteren, in Fig. 18A, 18B und 19A-19D dargestellten Ausführungsbeispiel ist ein Schaft 2 eines medizinischen Instruments 1 aus drei Schaftsegmenten 20, 21, 22 hergestellt, wobei die Schaftsegmente 20, 21, 22 über Gelenke 3A, 3B gelenkig miteinander verbunden sind. Zwei Verbindungselemente 4A, 4B bilden ein einstückiges Element und koppeln die Schaftsegmente 20, 21, 22 miteinander, wobei ein erstes Verbindungselement 4A gelenkig an ein längsverschieblich an dem ersten Schaftsegment 20 geführtes Verstellelement 5A angebunden und über eine Gelenkverbindung 41 gelenkig und an einem Langloch 210 längsverschieblich an dem zweiten Schaftsegment 21 angelenkt ist. Ein zweites Verbindungselement 4B erstreckt sich zwischen der Gelenkverbindung 41 und einer Gelenkverbindung 42, an der das Verbindungselement 4B gelenkig und ortsfest mit dem dritten Schaftsegment 22 verbunden ist.

Das erste Schaftsegment 20 und das dritte Schaftsegment 22 sind ähnlich dem Ausführungsbeispiel gemäß Fig. 13 bis 17 ausgestaltet und insbesondere abschnittsweise halbrohrförmig mit Aussparungen 204, 224 und Aufnahmeöffnungen 24 ausgebildet. Analog wie vorangehend beschrieben liegen das erste Schaftsegment 20 und das dritte Schaftsegment 22 in einem Längserstreckungszustand (siehe Fig. 19A) aneinander an und bilden eine näherungsweise geschlossene Hülle aus, in der das zweite Schaftsegment 21 und die Verbindungselemente 4A, 4B aufgenommen sind.

Das zweite Schaftsegment 21 erstreckt sich bei dem dargestellten Ausführungsbeispiel stabförmig und führt die durch einen Gelenkbolzen ausgeführte Gelenkverbindung 41 längsverschieblich an dem Langloch 210. Das zweite Schaftsegment 21 weist Verbindungsstücke 216, 217 auf, über die das zweite Schaftsegment 21 gelenkig mit dem ersten Schaftsegment 20 und dem dritten Schaftsegment 22 verbunden ist.

Bei dem dargestellten Ausführungsbeispiel weisen die Verbindungselemente 4A, 4B deutlich voneinander abweichende Längen auf. Entsprechend ergibt sich bei einem Verstellen des Schafts 2 aus seinem Längserstreckungszustand (Fig. 19A) heraus die in Fig. 19B-19D dargestellte Bewegung, infolge derer bei einem Verstellen des Verstellelements 5A in die Verstellrichtung V das zweite Schaftsegment 21 ausgeschwenkt und das dritte Schaftsegment 22 von dem ersten Schaftsegment 20 entfernt wird. Hierbei wird das dritte Schaftsegment 22 zunächst mit seinem vom Gelenk 3B abliegenden Ende vom ersten Schaftsegment 20 weg verschwenkt, wobei sich jedoch bei weiterer Bewegung die Schwenkrichtung des dritten Schaftsegments 22 umkehrt und durch verstärkte Aufstellung des zweiten Schaftsegments 21 bei sich nur noch unwesentlich ändernder Winkellage der Verbindungselemente 4A, 4B das von dem Gelenk 3B abliegende Ende des dritten Schaftsegments 22 wiederum der Längsachse L, entlang derer sich das erste Schaftsegment 20 erstreckt, angenähert wird. Es ergibt sich in einem verschwenkten Zustand die in Fig. 19D dargestellte Lage des dritten Schaftsegments 22, das in dem verschwenkten Zustand mit seinem vom Gelenk 3B abliegenden Ende hin zur Längsachse L des ersten Schaftsegments 20 weist.

Bei dem in Fig. 18A, 18B und 19A-19D dargestellten Ausführungsbeispiel ist das zweite Verbindungselement 4B über die Gelenkverbindung 42 gelenkig, aber ortsfest mit dem dritten Schaftsegment 22 verbunden. Das zweite Schaftsegment 21 führt die Gelenkverbindung 41 der Verbindungselemente 4A, 4B längsverschieblich entlang der Längserstreckungsrichtung des zweiten Schaftsegments 21, so dass auf diese Weise ein Längenausgleich bei der Verschwenkbewegung des zweiten Schaftsegments 21 geschaffen wird.

Im Längserstreckungszustand gemäß Fig. 19A nimmt der Schaft 2 eine kompakte Form ein, wobei, wie vorangehend bereits beschrieben, das zweite Schaftsegment 21 und die Verbindungselemente 4A, 4B zwischen dem ersten Schaftsegment 20 und dem dritten Schaftsegment 22 einliegen und somit von diesem eingehüllt sind. Die Verbindungsstücke 216, 217 des zweiten Schaftsegments 21 kommen hierbei in Aussparungen 46, 47 der Verbindungselemente 4A, 4B zu liegen. Die Verbindungselemente 4A, 4B liegen im Längserstreckungszustand in den Aufnahmeöffnungen 24 des ersten Schaftsegments 20 und des dritten Schaftsegments 22 ein und sind auf diese Weise eingefasst. Durch Verstellen des Verstellelements 5A in die Verstellrichtung V in dem ersten Schaftsegment 20 können die Verbindungselemente 4A, 4B und das zweite Schaftsegment 21 und damit auch das dritte Schaftsegment 22 aus dem ersten Schaftsegment 20 herausgestellt und verschwenkt werden.

Wiederum können Übertragungselemente für ein am dritten Schaftsegment 22 angeordnetes Werkzeug innerhalb des ersten Schaftsegments 20 und des dritten Schaftsegments 22 geführt werden, wobei solche Übertragungselemente vorzugsweise innerhalb der Verbindungselemente 4A, 4B vom ersten Schaftsegment 20 hin zum dritten Schaftsegment 22 verlegt werden, um auf diese Weise eine geschützte Führung für die Übertragungselemente innerhalb des Schafts 2 bereitzustellen.

Ein medizinisches Werkzeug der in diesem Text beschriebenen Art eignet sich insbesondere als Endoskop zur Durchführung eines medizinischen Eingriffs, insbesondere für einen sogenannten "Single Port"-Zugriff durch einen einzigen Port auf einen Operationsraum in einem Patienten. Durch die Abstützung der Schaftsegmente zueinander über eine oder mehrere Verbindungselemente wird eine stabile Anordnung geschaffen, die auch zur Übertragung vergleichsweise großer Betätigungskräfte geeignet ist. Die Schaftsegmente können beispielsweise in motorischer Weise zueinander verstellt werden und sind dadurch durch einen Nutzer komfortabel zu bedienen.

Medizinische Instrumente der hier beschriebenen Art sind insbesondere im Rahmen der Endoskopie bei minimal-invasiven Eingriffen verwendbar. Denkbar sind grundsätzlich aber auch andere Einsatzgebiete, gegebenenfalls auch außerhalb der Medizin, bei denen ein mehrere Schaftsegmente aufweisender Schaft in gelenkiger, schwenkbarer Weise hin zu einem Einsatzort gebracht werden soll.

Der Schaft kann grundsätzlich auch mehr als drei Segmente, beispielsweise vier oder fünf Segmente aufweisen, die in gekoppelter Weise oder auch unabhängig voneinander durch Einsatz geeigneter Verbindungselemente der vorangehend beschriebenen Art zueinander verschwenkt werden können.

### Bezugszeichenliste

- 1: Instrument
- 2: Schaft
- 20, 21, 22: Schaftsegment
- 200, 210, 220: Langloch
- 201, 211: Anschlag
- 212,222: Anschlag
- 213, 223: Schaftabschnitt
- 214,215: Anschlag
- 204, 224: Aussparung
- 216, 217: Verbindungsstück
- 218, 219: Aufnahmeöffnung
- 23: Innere Anlagefläche
- 230: Aussparung
- 24: Aufnahmeöffnung
- 3, 3A, 3B: Gelenk
- 4, 4A, 4B: Verbindungselement
- 40, 41, 40A, 40B, 41A, 41B, 42: Gelenkverbindung (Zapfen)
- 43: Innere Anlagefläche
- 430: Aussparung
- 44, 45: Ende
- 46,47: Aussparung
- 48: Hebelelement
- 480: Gelenk
- 5, 5A, 5B: Verstellelement (Rohrstück)
- 50: Betätigungsstange
- 51: Führungsabschnitt
- 6: Öffnung
- A: Abstand
- E: Schwenkebene
- L: Linie
- O: Operationsort
- V, V': Verstellrichtung
- Y: Querrichtung

## Patentansprüche

1. Medizinisches Instrument (1) zum Führen eines Werkzeugs in einen Operationsraum, mit
- einem Schaft (2), der ein erstes Schaftsegment (20, 21, 22) und ein zweites Schaftsegment (20, 21, 22), die gelenkig (3, 3A, 3B) miteinander verbunden sind, aufweist, und
- einem Verstellelement (5, 5A, 5B), das entlang einer Verstellrichtung (V) längsverschieblich in einer inneren Bohrung des ersten oder zweiten Schaftsegments (20, 21, 22) geführt und verstellbar ist, um das erste Schaftsegment (20, 21, 22) und das zweite Schaftsegment (20, 21, 22) relativ zueinander zu verschwenken, und
- einem Verbindungselement (4, 4A, 4B), das über eine erste Gelenkverbindung (40, 41, 40A, 40B, 41A, 41B, 42) gelenkig mit dem Verstellelement (5, 5A) und über eine zweite Gelenkverbindung (40, 41, 40A, 40B, 41A, 41B, 42) gelenkig mit dem anderen von erstem und zweitem Schaftsegment (20, 21, 22) verbunden ist,
wobei durch Verstellen des Verstellelements (5, 5A, 5B) entlang der Verstellrichtung (V) das erste Schaftsegment (20, 21, 22) und das zweite Schaftsegment (20, 21, 22) zueinander verschwenkbar sind,
wobei das Verstellelement (5, 5A, 5B) ausgebildet ist, das erste Schaftsegment (20, 21, 22) und das zweite Schaftsegment (20, 21, 22) zwischen einem Längserstreckungszustand, in dem das erste Schaftsegment (20, 21, 22) und das zweite Schaftsegment (20, 21, 22) im Wesentlichen kollinear miteinander fluchten, und einem verschwenkten Zustand, in dem das erste Schaftsegment (20, 21, 22) und das zweite Schaftsegment (20, 21, 22) aus dem Längserstreckungszustand verschwenkt sind, zu verschwenken,
**dadurch gekennzeichnet,**
**dass** das Verbindungselement (4, 4A, 4B) als ein halbzylindrisch geformtes Halbrohr ausgebildet ist und eine innere Anlagefläche (43) aufweist, an dem in dem Längserstreckungszustand zumindest eines von erstem und zweitem Schaftsegment (20, 21, 22) anliegt.

2. Medizinisches Instrument nach Anspruch 1, **dadurch gekennzeichnet, dass** in dem verschwenkten Zustand die innere Anlagefläche (43) außer Anlage mit dem ersten und zweiten Schaftsegment (20, 21, 22) ist.

3. Medizinisches Instrument nach einem der vorangehenden Ansprüche, **dadurch gekennzeichnet, dass** die erste Gelenkverbindung (40, 41, 40A, 40B, 41A, 41B, 42) und die zweite Gelenkverbindung (40, 41, 40A, 40B, 41A, 41B, 42) in dem Längserstreckungszustand auf einer Linie (L) liegen und ein Gelenk (3, 3A, 3B), das das erste Schaftsegment (20, 21, 22) und das zweite Schaftsegment (20, 21, 22) gelenkig miteinander verbindet, quer zu der Linie um einen Abstand (A) von der Linie (L) beabstandet ist.

4. Medizinisches Instrument nach einem der vorangehenden Ansprüche, **dadurch gekennzeichnet, dass** die erste Gelenkverbindung (40, 41, 40A, 40B, 41A, 41B, 42) ein entlang der Verstellrichtung (V) erstrecktes Langloch (200, 210, 220) des zugeordneten Schaftsegments (20, 21, 22) durchgreift.

5. Medizinisches Instrument nach einem der vorangehenden Ansprüche, **dadurch gekennzeichnet, dass** der Schaft (2) ein weiteres, drittes Schaftsegment (20, 21, 22) aufweist, das gelenkig mit dem zweiten Schaftsegment (20, 21, 22) verbunden ist, wobei ein weiteres, zweites Verbindungselement (4, 4A, 4B) zwischen dem zweiten Schaftsegment (20, 21, 22) und dem dritten Schaftsegment (20, 21, 22) wirkt.

6. Medizinisches Instrument nach Anspruch 5, **dadurch gekennzeichnet, dass** das erste Schaftsegment (20, 21, 22), das zweite Schaftsegment (21) und das dritte Schaftsegment (20, 21, 22) in einem Längserstreckungszustand im Wesentlichen kollinear zueinander angeordnet sind und in einem verschwenkten Zustand in einer gemeinsamen Schwenkebene (E) zueinander verschwenkt sind.

7. Medizinisches Instrument nach Anspruch 5 oder 6, **dadurch gekennzeichnet, dass** ein das erste Schaftsegment (20, 21, 22) und das zweite Schaftsegmente (20, 21, 22) verbindendes, erstes Gelenk (3, 3A, 3B) und ein das zweite Schaftsegment (20, 21, 22) und das dritte Schaftsegmente (20, 21, 22) verbindendes, zweites Gelenk (3, 3A, 3B) in dem Längserstreckungszustand in unterschiedliche Richtungen quer zu einer längs entlang der Schaftsegmente (20, 21, 22) erstreckten Mittenlinie (L) versetzt sind.

8. Medizinisches Instrument nach einem der Ansprüche 5 bis 7, **dadurch gekennzeichnet, dass** das zweite Verbindungselement (4, 4A, 4B) getrennt von dem zwischen dem ersten Schaftsegment (20, 21, 22) und dem zweiten Schaftsegment (20, 21, 22) wirkenden, ersten Verbindungselement (4, 4A, 4B) ausgebildet ist und über eine dritte Gelenkverbindung (40, 41, 40A, 40B, 41A, 41B, 42) mit einem von zweitem und drittem Schaftsegment (21, 22) und über eine vierte Gelenkverbindung (40, 41, 40A, 40B, 41A, 41B, 42) mit einem an dem anderen von zweitem und drittem Schaftsegment (21, 22) verstellbar angeordneten, zweiten Verstellelement (5, 5A, 5B) verbunden ist.

9. Medizinisches Instrument nach einem der Ansprüche 5 bis 7, **dadurch gekennzeichnet, dass** das zweite Verbindungselement (4, 4A, 4B) starr mit dem zwischen dem ersten Schaftsegment (20, 21, 22) und dem zweiten Schaftsegment (20, 21, 22) wirkenden, ersten Verbindungselement (4, 4A, 4B) ausgebildet ist.

10. Medizinisches Instrument nach Anspruch 9, **dadurch gekennzeichnet, dass** das erste Verbindungselement (4, 4A, 4B) und zweite Verbindungselement (4, 4A, 4B) einstückig ausgebildet sind.

11. Medizinisches Instrument nach Anspruch 9 oder 10, **dadurch gekennzeichnet, dass** ein erstes Verstellelement (5, 5A, 5B) verstellbar an dem ersten Schaftsegment (20, 21, 22) und ein zweites Verstellelement (5, 5A, 5B) verstellbar an einem von zweitem und drittem Schaftsegment (20, 21, 22) angeordnet ist, wobei das erste Verbindungselement (4, 4A, 4B) über die erste Gelenkverbindung (40, 41, 42) gelenkig mit dem ersten Verstellelement (5, 5A, 5B) und über die zweite Gelenkverbindung (40, 41, 42) gelenkig mit dem zweiten Schaftsegment (20, 21, 22) oder dem zweiten Verstellelement (5, 5A, 5B) und das zweite Verbindungselement (4, 4A, 4B) über die zweite Gelenkverbindung (40, 41, 42) gelenkig mit dem zweiten Schaftsegment (20, 21, 22) oder dem zweiten Verstellelement (5, 5A, 5B) und über eine dritte Gelenkverbindung (40, 41, 42) gelenkig mit dem zweiten Verstellelement (5, 5A, 5B) oder dem dritten Schaftsegment (20, 21, 22) verbunden ist.

12. Medizinisches Instrument nach Anspruch 11, **dadurch gekennzeichnet, dass** das erst Verstellelement (5A) oder das zweite Verstellelement (5B) motorisch angetrieben ist.

13. Medizinisches Instrument nach einem der Ansprüche 5 bis 12, **dadurch gekennzeichnet, dass** das zweite Schaftsegment (21) und/oder die Verbindungselemente (4, 4A, 4B) in dem Längserstreckungszustand zwischen dem ersten Schaftsegment (20) und dem dritten Schaftsegment (22) aufgenommen und zumindest abschnittsweise von dem ersten Schaftsegment (20) und dem dritten Schaftsegment (22) eingefasst sind.

14. Medizinisches Instrument nach einem der Ansprüche 5 bis 13, **dadurch gekennzeichnet, dass** das erste Schaftsegment (20) und das dritte Schaftsegment (22) jeweils eine Aufnahmeöffnung (24) aufweisen, in der das zweite Schaftsegment (21) und/oder die Verbindungselemente (4, 4A, 4B) in dem Längserstreckungszustand einliegen.

15. Medizinisches Instrument nach einem der Ansprüche 5 bis 14, **dadurch gekennzeichnet, dass** bei Verstellen des Schafts (2) aus seinem Längserstreckungszustand das dritte Schaftsegment (22) durch Verstellen des Verstellelements (4, 4A, 4B) entlang der Verstellrichtung (V) in eine quer zur Verstellrichtung (V) gerichtete Querrichtung (Y) von dem ersten Schaftsegment (20) entfernt wird.

16. Medizinisches Instrument nach einem der Ansprüche 5 bis 7, **dadurch gekennzeichnet, dass** das zweite Schaftsegment (21) und das Verbindungselement (4) jeweils als Halbrohr ausgebildet sind und in einem Längserstreckungszustand das erste Schaftsegment (20) und/oder das zweite Schaftsegment (22) zumindest abschnittsweise in sich aufnehmen, wobei das erste Schaftsegment (20, das zweite Schaftsegment (21), das dritte Schaftsegment (22) und das Verbindungselement (4) ein Viergelenk bilden.

17. Medizinisches Instrument nach Anspruch 16, **dadurch gekennzeichnet, dass** das zweite Schaftsegment (21) und das Verbindungselement (4) an unterschiedlichen, längs entlang des dritten Schaftsegments (22) zueinander versetzten Gelenkverbindungen (3B, 41) mit dem dritten Schaftsegment (22) gelenkig verbunden sind.

18. Medizinisches Instrument nach Anspruch 16 oder 17, **dadurch gekennzeichnet, dass** das Verbindungselement (4) gelenkig mit einem längsverschieblich am ersten Schaftsegment (20) geführten Verstellelement (5) verbunden ist, wobei ein Hebelelement (48) sich zwischen dem Verbindungselement (4) und dem ersten Schaftsegment (20) erstreckt und gelenkig mit dem Verbindungselement (4) und dem ersten Schaftsegment (20) verbunden ist.

## Claims

1. Medical instrument (1) for guiding a tool into an operating space, with
- a shaft (2), which has a first shaft segment (20, 21, 22) and a second shaft segment (20, 21, 22) connected to each other in an articulated manner (3, 3A, 3B), and
- an adjustment element (5, 5A, 5B), which is guided longitudinally movably in an inner bore of the first shaft segment or second shaft segment (20, 21, 22) and is adjustable along an adjustment direction (V), in order to pivot the first shaft segment (20, 21, 22) and the second shaft segment (20, 21, 22) relative to each other, and
- a connection element (4, 4A, 4B), which is connected in an articulated manner to the adjustment element (5, 5A) via a first hinge connection (40, 41, 40A, 40B, 41A, 41B, 42) and is connected in an articulated manner to the other of the first and second shaft segments (20, 21, 22) via a second hinge connection (40, 41, 40A, 40B, 41A, 41B, 42),
wherein, by adjusting the adjustment element (5, 5A, 5B) along the adjustment direction (V), the first shaft segment (20, 21, 22) and the second shaft segment (20, 21, 22) are pivotable relative to each other,
wherein the adjustment element (5, 5A, 5B) is designed to pivot the first shaft segment (20, 21, 22) and the second shaft segment (20, 21, 22) between a longitudinal extension state, in which the first shaft segment (20, 21, 22) and the second shaft segment (20, 21, 22) are aligned substantially collinearly with each other, and a pivoted state, in which the first shaft segment (20, 21, 22) and the second shaft segment (20, 21, 22) are pivoted from the longitudinal extension state.
**characterized**
**in that** the connection element (4, 4A, 4B) is designed as a semicylindrically shaped half-tube and has an inner bearing surface (43), on which at least one of the first and second shaft segments (20, 21, 22) bears in the longitudinal extension state.

2. Medical instrument according to Claim 1, **characterized in that,** in the pivoted state, the inner bearing surface (43) does not bear on the first and second shaft segments (20, 21, 22).

3. Medical instrument according to one of the preceding claims, **characterized in that** the first hinge connection (40, 41, 40A, 40B, 41A, 41B, 42) and the second hinge connection (40, 41, 40A, 40B, 41A, 41B, 42) lie on a line (L) in the longitudinal extension state, and a hinge (3, 3A, 3B), which connects the first shaft segment (20, 21, 22) and the second shaft segment (20, 21, 22) to each other in an articulated manner, is arranged, transversely with respect to the line, at a distance (A) from the line (L).

4. Medical instrument according to one of the preceding claims, **characterized in that** the first hinge connection (40, 41, 40A, 40B, 41A, 41B, 42) engages through an oblong hole (200, 210, 220) of the associated shaft segment (20, 21, 22), which oblong hole (200, 210, 220) extends along the adjustment direction (V).

5. Medical instrument according to one of the preceding claims, **characterized in that** the shaft (2) has a further, third shaft segment (20, 21, 22), which is connected in an articulated manner to the second shaft segment (20, 21, 22), wherein a further, second connection element (4, 4A, 4B) acts between the second shaft segment (20, 21, 22) and the third shaft segment (20, 21, 22).

6. Medical instrument according to Claim 5, **characterized in that** the first shaft segment (20, 21, 22), the second shaft segment (21) and the third shaft segment (20, 21, 22) are arranged substantially collinearly to one another in a longitudinal extension state and are pivoted relative to one another in a common pivot plane (E) in a pivoted state.

7. Medical instrument according to Claim 5 or 6, **characterized in that** a first hinge (3, 3A, 3B), connecting the first shaft segment (20, 21, 22) and the second shaft segment (20, 21, 22), and a second hinge (3, 3A, 3B), connecting the second shaft segment (20, 21, 22) and the third shaft segment (20, 21, 22), are offset, in the longitudinal extension state, transversely in different directions with respect to a midline (L) extending along the shaft segments (20, 21, 22).

8. Medical instrument according to one of Claims 5 to 7, **characterized in that** the second connection element (4, 4A, 4B) is designed separately from the first connection element (4, 4A, 4B) acting between the first shaft segment (20, 21, 22) and the second shaft segment (20, 21, 22) and is connected, via a third hinge connection (40, 41, 40A, 40B, 41A, 41B, 42), to one of the second and third shaft segments (21, 22) and, via a fourth hinge connection (40, 41, 40A, 40B, 41A, 41B, 42), to a second adjustment element (5, 5A, 5B) arranged adjustably on the other of the second and third shaft segments (21, 22).

9. Medical instrument according to one of Claims 5 to 7, **characterized in that** the second connection element (4, 4A, 4B) is designed rigidly with the first connection element (4, 4A, 4B) acting between the first shaft segment (20, 21, 22) and the second shaft segment (20, 21, 22).

10. Medical instrument according to Claim 9, **characterized in that** the first connection element (4, 4A, 4B) and second connection element (4, 4A, 4B) are designed in one piece.

11. Medical instrument according to Claim 9 or 10, **characterized in that** a first adjustment element (5, 5A, 5B) is arranged adjustably on the first shaft segment (20, 21, 22) and a second adjustment element (5, 5A, 5B) is arranged adjustably on one of the second and third shaft segments (20, 21, 22), wherein the first connection element (4, 4A, 4B) is connected in an articulated manner to the first adjustment element (5, 5A, 5B) via the first hinge connection (40, 41, 42) and is connected in an articulated manner to the second shaft segment (20, 21, 22) or the second adjustment element (5, 5A, 5B) via the second hinge connection (40, 41, 42), and the second connection element (4, 4A, 4B) is connected in an articulated manner to the second shaft segment (20, 21, 22) or the second adjustment element (5, 5A, 5B) via the second hinge connection (40, 41, 42) and is connected in an articulated manner to the second adjustment element (5, 5A, 5B) or the third shaft segment (20, 21, 22) via a third hinge connection (40, 41, 42) .

12. Medical instrument according to Claim 11, **characterized in that** the first adjustment element (5A) or the second adjustment element (5B) is driven by motor.

13. Medical instrument according to one of Claims 5 to 12, **characterized in that** the second shaft segment (21) and/or the connection elements (4, 4A, 4B) in the longitudinal extension state are received between the first shaft segment (20) and the third shaft segment (22) and are at least partially enclosed by the first shaft segment (20) and the third shaft segment (22).

14. Medical instrument according to one of Claims 5 to 13, **characterized in that** the first shaft segment (20) and the third shaft segment (22) each have a receiving opening (24), in which the second shaft segment (21) and/or the connection elements (4, 4A, 4B) lie in the longitudinal extension state.

15. Medical instrument according to one of Claims 5 to 14, **characterized in that,** upon adjustment of the shaft (2) from its longitudinal extension state, the third shaft segment (22), by adjustment of the adjustment element (5, 5A, 5B) along the adjustment direction (V), is moved away from the first shaft segment (20) in a transverse direction (Y) oriented transversely with respect to the adjustment direction (V).

16. Medical instrument according to one of Claims 5 to 7, **characterized in that** the second shaft segment (21) and the connection element (4) are each designed as a half-tube and, in a longitudinal extension state, at least partially receive the first shaft segment (20) and/or the second shaft segment (22), wherein the first shaft segment (20), the second shaft segment (21), the third shaft segment (22) and the connection element (4) form a four-bar linkage.

17. Medical instrument according to Claim 16, **characterized in that** the second shaft segment (21) and the connection element (4) are connected in an articulated manner to the third shaft segment (22) at different hinge connections (3B, 41) offset relative to each other along the third shaft segment (22).

18. Medical instrument according to Claims 16 or 17 through 27, **characterized in that** the connection element (4) is connected in an articulated manner to an adjustment element (5) guided longitudinally movably on the first shaft segment (20), wherein a lever element (48) extends between the connection element (4) and the first shaft segment (20) and is connected in an articulated manner to the connection element (4) and the first shaft segment (20) .

## Revendications

1. Instrument médical (1) pour guider un outil dans une salle d'opération, comprenant
- un arbre (2) qui présente un premier segment d'arbre (20, 21, 22) et un deuxième segment d'arbre (20, 21, 22) qui sont reliés l'un à l'autre de manière articulée (3, 3A, 3B), et
- un élément de réglage (5, 5A, 5B) qui est guidé le long d'une direction de réglage (V) de manière déplaçable longitudinalement dans un alésage interne du premier ou du deuxième segment d'arbre (20, 21, 22) et qui peut être réglé pour faire pivoter le premier segment d'arbre (22, 21, 22) et le deuxième segment d'arbre (20, 21, 22) l'un par rapport à l'autre, et
- un élément de connexion (4, 4A, 4B) qui est connecté par le biais d'une première connexion articulée (40, 41, 40A, 40B, 41A, 41B, 42) de manière articulée à l'élément de réglage (5, 5A) et par le biais d'une deuxième connexion articulée (40, 41, 40A, 40B, 41A, 41B, 42) de manière articulée à l'autre parmi le premier et le deuxième segment d'arbre (20, 21, 22),
dans lequel, par réglage de l'élément de réglage (5, 5A, 5B) le long de la direction de réglage (V), le premier segment d'arbre (20, 21, 22) et le deuxième segment d'arbre (20, 21, 22) peuvent pivoter l'un par rapport à l'autre,
dans lequel l'élément de réglage (5, 5A, 5B) est réalisé de manière à faire pivoter le premier segment d'arbre (20, 21, 22) et le deuxième segment d'arbre (20, 21, 22) entre un état d'étendue longitudinale dans lequel le premier segment d'arbre (20, 21, 22) et le deuxième segment d'arbre (20, 21, 22) sont essentiellement en affleurement colinéaire l'un avec l'autre, et un état pivoté dans lequel le premier segment d'arbre (20, 21, 22) et le deuxième segment d'arbre (20, 21, 22) sont pivotés hors de l'état d'étendue longitudinale,
**caractérisé en ce que**
l'élément de connexion (4, 4A, 4B) est réalisé sous forme de demi-tube de forme semi-cylindrique et présente une surface d'appui intérieure (43) contre laquelle s'applique au moins l'un parmi le premier et le deuxième segment d'arbre (20, 21, 22) dans l'état d'étendue longitudinale.

2. Instrument médical selon la revendication 1, **caractérisé en ce que** dans l'état pivoté, la surface d'appui intérieure (43) n'est pas en appui contre le premier et le deuxième segment d'arbre (20, 21, 22).

3. Instrument médical selon l'une quelconque des revendications précédentes, **caractérisé en ce que** la première connexion articulée (40, 41, 40A, 40B, 41A, 41B, 42) et la deuxième connexion articulée (40, 41, 40A, 40B, 41A, 41B, 42), dans l'état d'étendue longitudinale, sont situées sur une ligne (L) et une articulation (3, 3A, 3B) qui relie de manière articulée le premier segment d'arbre (20, 21, 22) et le deuxième segment d'arbre (20, 21, 22) l'un à l'autre est espacée de la ligne (L) d'une distance (A) transversalement à la ligne.

4. Instrument médical selon l'une quelconque des revendications précédentes, **caractérisé en ce que** la première connexion articulée (40, 41, 40A, 40B, 41A, 41B, 42) s'engage à travers un trou oblong (200, 210, 220) du segment d'arbre associé (20, 21, 22) s'étendant le long de la direction de réglage (V).

5. Instrument médical selon l'une quelconque des revendications précédentes, **caractérisé en ce que** l'arbre (2) présente un troisième segment d'arbre supplémentaire (20, 21, 22) qui est connecté de manière articulée au deuxième segment d'arbre (20, 21, 22), un deuxième élément de connexion supplémentaire (4, 4A, 4B) agissant entre le deuxième segment d'arbre (20, 21, 22) et le troisième segment d'arbre (20, 21, 22).

6. Instrument médical selon la revendication 5, **caractérisé en ce que** le premier segment d'arbre (20, 21, 22), le deuxième segment d'arbre (21) et le troisième segment d'arbre (20, 21, 22) sont disposés de manière essentiellement colinéaire les uns par rapport aux autres dans un état d'étendue longitudinale, et sont pivotés les uns par rapport aux autres dans un plan commun de pivotement (E) dans un état pivoté.

7. Instrument médical selon la revendication 5 ou 6, **caractérisé en ce qu'**une première articulation (3, 3A, 3B) reliant le premier segment d'arbre (20, 21, 22) et le deuxième segment d'arbre (20, 21, 22) et une deuxième articulation (3, 3A, 3B) reliant le deuxième segment d'arbre (20, 21, 22) et le troisième segment d'arbre (20, 21, 22), dans l'état d'étendue longitudinale, sont décalées dans des directions différentes transversalement à un axe médian (L) s'étendant longitudinalement le long des segments d'arbre (20, 21, 22).

8. Instrument médical selon l'une quelconque des revendications 5 à 7, **caractérisé en ce que** le deuxième élément de connexion (4, 4A, 4B) est réalisé séparément du premier élément de connexion (4, 4A, 4B) agissant entre le premier segment d'arbre (20, 21, 22) et le deuxième segment d'arbre (20, 21, 22) et est connecté par le biais d'une troisième connexion articulée (40, 41, 40A, 40B, 41A, 41B, 42) à l'un parmi le deuxième et le troisième segment d'arbre (21, 22) et par le biais d'une quatrième connexion articulée (40, 41, 40A, 40B, 41A, 41B, 42) à un deuxième élément de réglage (5, 5A, 5B) disposé de manière réglable au niveau de l'autre parmi le deuxième et le troisième segment d'arbre (21, 22).

9. Instrument médical selon l'une quelconque des revendications 5 à 7, **caractérisé en ce que** le deuxième élément de connexion (4, 4A, 4B) est réalisé rigidement avec le premier élément de connexion (4, 4A, 4B) agissant entre le premier segment d'arbre (20, 21, 22) et le deuxième segment d'arbre (20, 21, 22).

10. Instrument médical selon la revendication 9, **caractérisé en ce que** le premier élément de connexion (4, 4A, 4B) et le deuxième élément de connexion (4, 4A, 4B) sont réalisés d'une seule pièce.

11. Instrument médical selon la revendication 9 ou 10, **caractérisé en ce qu'**un premier élément de réglage (5, 5A, 5B) est disposé de manière réglable au niveau du premier segment d'arbre (20, 21, 22) et un deuxième élément de réglage (5, 5A, 5B) est disposé de manière réglable au niveau de l'un parmi le deuxième et le troisième segment d'arbre (20, 21, 22), le premier élément de connexion (4, 4A, 4B) étant connecté par le biais de la première connexion articulée (40, 41, 42) de manière articulée au premier élément de réglage (5, 5A, 5B) et par le biais de la deuxième connexion articulée (40, 41, 42) de manière articulée au deuxième segment d'arbre (20, 21, 22) ou au deuxième élément de réglage (5, 5A, 5B) et le deuxième élément de connexion (4, 4A, 4B) étant connecté par le biais de la deuxième connexion articulée (40, 41, 42) de manière articulée au deuxième segment d'arbre (20, 21, 22) ou au deuxième élément de réglage (5, 5A, 5B) et par le biais d'une troisième connexion articulée (40, 41, 42) de manière articulée au deuxième élément de réglage (5, 5A, 5B) ou au troisième segment d'arbre (20, 21, 22).

12. Instrument médical selon la revendication 11, **caractérisé en ce que** le premier élément de réglage (5A) ou le deuxième élément de réglage (5B) sont entraînés par un moteur.

13. Instrument médical selon l'une quelconque des revendications 5 à 12, **caractérisé en ce que** le deuxième segment d'arbre (21) et/ou les éléments de connexion (4, 4A, 4B), dans l'état d'étendue longitudinale, sont reçus entre le premier segment d'arbre (20) et le troisième segment d'arbre (22) et sont encadrés au moins en partie par le premier segment d'arbre (20) et par le troisième segment d'arbre (22).

14. Instrument médical selon l'une quelconque des revendications 5 à 13, **caractérisé en ce que** le premier segment d'arbre (20) et le troisième segment d'arbre (22) présentent chacun une ouverture de réception (24) dans laquelle le deuxième segment d'arbre (21) et/ou les éléments de connexion (4, 4A, 4B) sont insérés dans l'état d'étendue longitudinale.

15. Instrument médical selon l'une quelconque des revendications 5 à 14, **caractérisé en ce que** lors du réglage de l'arbre (2) hors de son état d'étendue longitudinale, le troisième segment d'arbre (22) est éloigné du premier segment d'arbre (20) par réglage de l'élément de réglage (5, 5A, 5B) le long de la direction de réglage (V) dans une direction transversale (Y) orientée transversalement par rapport à la direction de réglage (V).

16. Instrument médical selon l'une quelconque des revendications 5 à 7, **caractérisé en ce que** le deuxième segment d'arbre (21) et l'élément de connexion (4) sont chacun réalisés sous forme de demi-tube et reçoivent en eux, au moins en partie, dans un état d'étendue longitudinale le premier segment d'arbre (20) et/ou le deuxième segment d'arbre (22), le premier segment d'arbre (20), le deuxième segment d'arbre (21), le troisième segment d'arbre (22) et l'élément de connexion (4) formant un quadrilatère articulé.

17. Instrument médical selon la revendication 16, **caractérisé en ce que** le deuxième segment d'arbre (21) et l'élément de connexion (4) sont connectés de manière articulée au troisième segment d'arbre (22) au niveau de connexions articulées différentes (3B, 41) décalées les unes par rapport aux autres longitudinalement le long du troisième segment d'arbre (22).

18. Instrument médical selon la revendication 16 ou 17, **caractérisé en ce que** l'élément de connexion (4) est connecté de manière articulée à un élément de réglage (5) guidé de manière déplaçable longitudinalement au niveau du premier segment d'arbre (20), un élément de levier (48) s'étendant entre l'élément de connexion (4) et le premier segment d'arbre (20) et étant connecté de manière articulée à l'élément de connexion (4) et au premier segment d'arbre (20).
